# EUROPÄISCHE PATENTSCHRIFT

(11) **EP 3 679 013 B1**
(45) Veröffentlichungstag und Bekanntmachung des Hinweises auf die Patenterteilung: **29.11.2023**
(21) Anmeldenummer: 18759141.7
(22) Anmeldetag: 31.08.2018
(51) Int. Cl.: C07C 263/04, C07C 265/12, C07C 265/14, C07C 265/04

(54) **VERFAHREN ZUR HERSTELLUNG VON ISOCYANATEN**
METHOD FOR MAKING ISOCYANATES
PROCÉDÉ DE FABRICATION D'ISOCYANATES

(30) Priorität: 04.09.2017 EP 17189248
(43) Veröffentlichungstag der Anmeldung: 15.07.2020
(73) Patentinhaber: Covestro Intellectual Property GmbH & Co. KG, 51373 Leverkusen (DE)
(72) Erfinder: COZZULA, Daniela, 53562 Sankt Katharinen (DE); ERNST, Andreas, 52062 Aachen (DE); LEVEN, Matthias, 51069 Köhl (DE); LEITNER, Walter, 52074 Aachen (DE); MÜLLER, Thomas Ernst, 44797 Bochum (DE); GÜRTLER, Christoph, 50735 Köln (DE); WERSHOFEN, Stefan, 41065 Mönchengladbach (DE); JÄGER, Gernot, 50733 Köln (DE); BEGGEL, Franz, 50935 Köln (DE); LANGANKE, Jens, 53894 Mechernich (DE)
(74) Vertreter: Levpat
(86) Internationale Anmeldenummer: PCT/EP2018/073512
(87) Internationale Veröffentlichungsnummer: WO 2019/043180

(56) Entgegenhaltungen:
- EP-A1- 0 672 653
- US-A- 4 081 472
- US-A1- 2016 145 201
- DATABASE WPI Week 201161 Thomson Scientific, London, GB; AN 2011-K77649 XP002778483, & JP 2011 162442 A (MITSUI CHEM INC) 25. August 2011 (2011-08-25) in der Anmeldung erwähnt
- DATABASE CAPLUS [Online] CHEMICAL ABSTRACTS SERVICE, COLUMBUS, OHIO, US; KIM, SAM-MIN ET AL: "Process for the preparation of organic isocyanates using 2-halopyridium salts and aromatic or aliphatic (methylthio)", XP002778484, gefunden im STN Database accession no. 2000:648825 in der Anmeldung erwähnt & KR 970 004 412 B1 (KOREA KUMHO PETROCHEM CO LTD [KR]) 27. März 1997 (1997-03-27)

## Beschreibung

Die Erfindung betrifft ein Verfahren zur Herstellung eines Isocyanats, bei welchem ein Carbamat oder Thiolcarbamat in Gegenwart eines Katalysators unter Abspaltung eines Alkohols oder Thioalkohols bei einer Temperatur von mindestens 150 °C zu dem korrespondierenden Isocyanat umgesetzt wird, wobei als Katalysator eine Verbindung der allgemeinen Formel (X)(Y)(Z-H) eingesetzt wird, die insbesondere dadurch gekennzeichnet ist, dass sie sowohl über eine Protonendonatorfunktion als auch über eine Protonenaktzeptorfunktion verfügt. In den erfindungsgemäßen Katalysatoren ist ein abspaltbares Proton an ein Heteroatom, das elektronegativer als Kohlenstoff ist, gebunden. Dieses Heteroatom ist entweder identisch mit Z oder Bestandteil desselben. In den erfindungsgemäßen Katalysatoren ist ferner eine Protonenakzeptorfunktion enthalten, die entweder identisch mit X oder ein Bestandteil desselben ist. Protonendonator- und Protonenakzeptorfunktion sind über die Brücke Y miteinander verbunden.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Die Umsetzung der Amine mit dem Phosgen kann sowohl in der Gasphase als auch in der Flüssigphase erfolgen, wobei die Reaktion diskontinuierlich oder kontinuierlich durchgeführt werden kann. Es kommen dabei sowohl aromatische Isocyanate wie beispielsweise Methylendiphenyldiisocyanat (MMDI - "monomeres MDI"), Polymethylen-Polyphenylen-Polyisocyanat (ein Gemisch aus MMDI und höheren Homologen, PMDI, "polymeres MDI") oder Toluylendiisocyanat (TDI) als auch aliphatische Isocyanate wie z. B. Pentandiisocyanat (PDI), Hexamethylendiisocyanat (HDI) oder Isophorondiisocyanat (IPDI) weltweit zum Einsatz.

Eine Alternative zur Umsetzung von primären Aminen mit Phosgen stellt die Carbamatspaltung dar:

R-NH-(C=O)-O-R' -> R-N=C=O + H-O-R'

R und R' bezeichnen organische Reste. Es ist ebenfalls möglich, *Thiol*carbamate R-NH-(C=O)-S-R' unter Abspaltung eines *Thio*alkohols H-S-R' in Isocyanate zu überführen.

Die Carbamatspaltung kann thermisch erfolgen oder durch Katalysatoren bzw. stöchiometrisch eingesetzte Hilfsreagenzien vermittelt werden. Die thermische Spaltung findet im Allgemeinen oberhalb einer Temperatur von ca. 180 °C statt. Als Katalysatoren bzw. Hilfsreagenzien für die katalytische Carbamatspaltung wurden die verschiedensten Verbindungsklassen beschrieben. Einige Beispiele sind im Folgenden genannt:
Eine Veröffentlichung in Tetrahedron Letters (43), 2002, 1673-1676 (P. Uriz et al.) beschäftigt sich mit der Verwendung des Schichtsilikats Montmorillonit K10 als Katalysator für die Carbamatspaltung. Es wird dabei die Hypothese vertreten, dass die Carbamate über die vorhandenen Brønstedsäurezentren protoniert und durch anschließende Umprotonierung zum Isocyanat und Alkohol gespalten werden.

CN 000101337189 beschreibt ebenfalls die Verwendung von Feststoffsäuren der Art SO42-/TiO₂-ZnO-ZrO₂-Al₂O₃ hergestellt aus Titanaten (8 bis 45 mol-%), wasserlöslichen Zinksalzen (30 bis 60 mol-%, wasserlöslichen Aluminiumsalzen (3 bis 10 mol-%, wasserlöslichen Zirkoniumsalzen (8 bis 20 mol-% und sowie Schwefelsäure (6 bis 18 mol-%).

Eine Veröffentlichung in J. Org. Chem. (63), 2000, 3239-3240 (S. Gastaldi et al.) beschreibt die Verwendung von Diisopropylethylamin ("Hünig-Base") und SiI₂H₂ in der Carbamatspaltung. Beide werden dabei stöchiometrisch eingesetzt.

In der Patentanmeldung EP 0 672 653 A1 wird die Herstellung von Isocyanaten durch Carbamatspaltung bei 150 °C bis 350 °C in Gegenwart von organischen Sulfonsäuren des Typs R¹SO₃H oder deren Salzen beschrieben. R¹ ist dabei ein organischer Rest, der mit Gruppen, die mit Isocyanaten nicht reagieren, substituiert sein kann, z. B. Halogen,- Alkoxy- oder Nitrogruppen. Als konkrete Beispiele werden mehrere aromatische (z. B. Naphthalin-*β*-sulfonsäure) und aliphatische (z. B. Methansulfonsäure) Sulfonsäuren sowie Alkalimetallsalze von aromatischen (Natrium-*meta-*xylol-4-sulfonat) und aliphatischen (Kaliummethansulfonat) Sulfonsäuren genannt. Katalysatoren mit einer Protonendonator- *und* Protonenakzeptorfunktion im Sinne der vorliegenden Erfindung werden nicht offenbart. Insbesondere offenbart diese Anmeldung nicht, aromatische oder araliphatische Disulfonsäuren oder die Monoanionen solcher Disulfonsäuren als Katalysatoren einzusetzen.

Die japanische Patentanmeldung JP 2011/162442 befasst sich ebenfalls mit Carbamatspaltung. Als Katalysatoren werden die Metallsalze nicht koordinierender Anionen offenbart. Als nicht koordinierende Anionen werden Perfluoroalkylsulfonat, Arylsulfonat, Hexafluorophosphat, Tetrafluoroborat, Tetrakis(pentafluorophenyl)-borat sowie Tetrakis-[3,5-bis(trifluoromethyl)-phenyl]-borat offenbart. Bevorzugt sind Arylsulfonsäureanionen, insbesondere CH₃C₆H₄SO₃⁽⁻⁾ (Toluolsulfonat). Katalysatoren mit einer zusätzlichen Protonendonatorfunktion werden nicht offenbart. Insbesondere fehlt es an einer Offenbarung von Monoanionen von aromatischen oder araliphatischen Disulfonsäuren als geeignete Katalysatoren.

Die koreanische Anmeldung mit der Nummer 19930018854 (auch veröffentlicht als KR970004412) beschreibt die Herstellung von Isocyanaten R'-NCO aus Thioncarbamaten (eine Untergruppe der Thiocarbamate; siehe die Erläuterungen zu Schema 1 weiter unten) und (stöchiometrisch eingesetzten) Halogenpyridiniumsalzen in Gegenwart von tertiären Aminen wie Triethylamin, Pyridin, Chinolin, Chinoxalin, Hexamethylentetramin, 1,4-Diazabicyclo[2.2.2]octan, 1,5-Diazabicyclo[4.3.0]non-5-en und 1,8-Diazabicylco[5.4.0]undec-7-en gemäß der Gleichung: 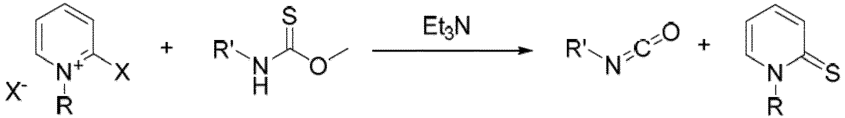 (X = F, Cl, I; R = Alkylrest; R' = aliphatischer oder aromatischer Rest)

Der Gegenstand der vorliegenden Erfindung, die Bildung von Isocyanaten durch Spaltung von Carbamaten oder Thiolcarbamaten (die beide eine -NH-(C=O)-Struktureinheit aufweisen) unter Freisetzung von Alkoholen bzw. Thioalkoholen, betrifft eine völlig andere chemische Reaktion. Es ist daher nicht verwunderlich, dass keiner der in dieser Schrift offenbarten tertiären AminKatalysatoren die Bedingungen eines Katalysators (X)(Y)(Z-H) der vorliegenden Erfindung erfüllt.

US 4,081,472 beschreibt die Durchführung von Carbamatspaltungen in Gegenwart von Metallionen der Gruppen I-B, II-B, III-A, IV-A, IV-B, V-B und VIII, insbesondere Kupfer-, Zink-, Aluminium-, Zinn-, Titan-, Vanadium-, Eisen-, Cobalt- und Nickel-Ionen. Besagte Metallionen werden u. a. als Salze von Carbonsäuren, als Alkhoholate oder Thioalkoholate, als Phenolate, Salze organischer Sulfonsäuren, als Chelat-Komplexe (bspw. von Acetylacetonat) und als Salze von Aminosäuren eingesetzt. Metallfreie Katalysatoren oder Katalysatoren, die Metalle allenfalls als katalytisch inaktives Gegenion enthalten, werden in dieser Schrift nicht offenbart.

US 2016/0145201 A1 beschreibt ein mehrstufiges Verfahren zur Herstellung von meta-Xylidendiisocyanat, bei dem anorganische Säuren wie Schwefel- oder Phosphorsäure Anwendung finden. Die Verwendung von Salzen oder organischen Estern solcher Säuren als Katalysatoren für die Spaltung von Carbamaten wird in dieser Schrift nicht offenbart.

Trotz Fortschritten auf dem Gebiet der phosgenfreien Isocyanat-Herstellung im Allgemeinen und der Carbamatspaltung im Besonderen besteht ein kontinuierlicher Verbesserungsbedarf dieser phosgenfreien Syntheserouten. Auch wenn die Carbamatspaltung unkatalysiert ablaufen kann, werden nach wie vor geeignete Katalysatoren gesucht, die die gewünschte Reaktion stark beschleunigen, ohne dass unerwünschte Nebenreaktionen und/oder Folgereaktionen in einem nennenswerten Umfang ablaufen, d. h. die eine hohe Selektivität der Isocyanatbildung aufweisen.

Diesem Bedarf Rechnung tragend ist ein Gegenstand der vorliegenden Erfindung ein Verfahren zur Herstellung eines Isocyanats, bei welchem ein Carbamat oder Thiolcarbamat in Gegenwart eines Katalysators unter Abspaltung eines Alkohols oder Thioalkohols zu dem korrespondierenden Isocyanat bei einer Temperatur von mindestens 150 °C umgesetzt wird, wobei als Katalysator eine Verbindung der allgemeinen Formel

**(X)(Y)(Z-H)**

eingesetzt wird, wobei gilt:
(A)
   - X steht für N(R¹),
   - Y steht für C(R²), eine Brücke aus 2 Kohlenstoffatomen, die Bestandteil eines Ringsystems aus 5 oder 7 Kohlenstoffatomen mit alternierenden Doppel- und Einfachbindungen sind, oder für eine Brücke aus 3, 5 oder 7 Kohlenstoffatomen mit alternierenden Einfach- und Doppelbindungen, und
   - Z steht für O, S, N(R⁶) oder N⁽⁺⁾(R⁷)(R⁸),
   - wobei die Katalysatoren des Typs (A) einen pK_{B}-Wert bei 25 °C von ≥ 3,00 aufweisen;
oder (B)
   - X steht für O,
   - Y steht für C(R²), eine Brücke aus 2 Kohlenstoffatomen, die Bestandteil eines Ringsystems aus 5 oder 7 Kohlenstoffatomen mit alternierenden Doppel- und Einfachbindungen sind, oder für eine Brücke aus 3, 5 oder 7 Kohlenstoffatomen mit alternierenden Einfach- und Doppelbindungen, und
   - Z steht für O;
oder (C)
   - X steht für O,
   - Y steht für S(O)(R³) oder P(OR⁴)(OR⁵), und
   - Z steht für O;
wobei gilt:
   R¹ ist
      - ein, optional substituierter, aromatischer oder araliphatischer Rest mit 6 bis 10 Kohlenstoffatomen oder
      - ein, optional substituierter, aliphatischer Rest mit 1 bis 6 Kohlenstoffatomen oder
      - mit R² oder R⁸ zu einem aus insgesamt 5 bis 8 Atomen bestehenden Ring verbunden, wobei der Ring optional Heteroatome, insbesondere Stickstoff und/oder Schwefel, umfassen kann;
   R² ist
      - Wasserstoff oder
      - ein, optional substituierter,aromatischer oder araliphatischer Rest mit 6 bis 10 Kohlenstoffatomen oder
      - ein, optional substituierter und optional Ethereinheiten aufweisender, aliphatischer Rest mit 1 bis 6 Kohlenstoffatomen oder
      - mit R¹ oder R⁶ oder R⁷ zu einem aus insgesamt 5 bis 8 Atomen bestehenden Ring verbunden, wobei der Ring optional Heteroatome, insbesondere Stickstoff und/oder Schwefel, umfassen kann;
   R³ ist
      - ein mit einer Sulfonsäure- oder Sulfonatgruppe substituierter, aromatischer oder araliphatischer Rest mit 6 bis 10 Kohlenstoffatomen oder
      - ein mit einer Amin-, Sulfonsäure- oder Sulfonatgruppe substituierter, aliphatischer Rest mit 1 bis 6 Kohlenstoffatomen oder
      - O⁽⁻⁾M⁽⁺⁾, wobei M⁽⁺⁾ für ein Alkalimetallkation, Imidazoliumkation, Pyridiniumkation, Pyrrolidiniumkation, Phosphoniumkation, Sulfoniumkation, NH₄⁺ oder für ein mono-, di-, tri- oder tetra-substituiertes organisches Ammoniumkation steht, dessen organische Substituenten unabhängig voneinander ausgewählt sind aus der Gruppe bestehend aus Methyl, Ethyl, Propyl, Butyl, Pentyl, Hexyl, Phenyl und Cyclohexyl, wobei besonders bevorzugt M⁽⁺⁾ für ein Alkalimetallkation oder NH₄⁺, ganz besonders bevorzugt für ein Alkalimetallkation ausgewählt aus Li⁺, Na⁺ oder K⁺, steht;
   R⁴ und R⁵ sind unabhängig voneinander
      - optional subsitituierte, aromatische oder araliphatische Reste mit jeweils 6 bis 10 Kohlenstoffatomen, wobei R⁴ und R⁵ zu einem aus 5 bis 8 Atomen bestehenden Ring verbunden sein können, oder
      - optional subsitituierte, aliphatische Reste mit jeweils 1 bis 6 Kohlenstoffatomen, wobei R⁴ und R⁵ zu einem aus 5 bis 8 Atomen bestehenden Ring verbunden sein können;
   R⁶ ist
      - ein, optional substituierter, aromatischer oder araliphatischer Rest mit 6 bis 10 Kohlenstoffatomen oder
      - ein, optional substituierter, aliphatischer Rest mit 1 bis 6 Kohlenstoffatomen oder
      - mit R² zu einem aus insgesamt 5 bis 8 Atomen bestehenden Ring verbunden, wobei der Ring optional Heteroatome, insbesondere Stickstoff und/oder Schwefel, umfassen kann;
   R⁷ ist
      - ein, optional substituierter, aromatischer oder araliphatischer Rest mit 6 bis 10 Kohlenstoffatomen oder
      - ein, optional substituierter, aliphatischer Rest mit 1 bis 6 Kohlenstoffatomen oder
      - mit R² zu einem aus insgesamt 5 bis 8 Atomen bestehenden Ring verbunden, wobei der Ring optional Heteroatome, insbesondere Stickstoff und/oder Schwefel, umfassen kann;
   R⁸ ist
      - ein, optional substituierter, aromatischer oder araliphatischer Rest mit 6 bis 10 Kohlenstoffatomen oder
      - ein, optional substituierter, aliphatischer Rest mit 1 bis 6 Kohlenstoffatomen oder mit R¹ zu einem aus insgesamt 5 bis 8 Atomen bestehenden Ring verbunden.

Erfindungsgemäß einsetzbare *Carbamate* haben die allgemeine Formel R-NH-(C=O)-O-R', worin R und R' organische Reste bezeichnen (besonders bevorzugte Reste R und R' werden weiter unten aufgeführt). Erfindungsgemäß werden unter *Thiolcarbamaten* (auch als *Thiolurethane* bezeichnet) Verbindungen des Typs R-NH-(C=O)-S-R' verstanden, bei denen eine S-Organylgruppe (S-R') an das Kohlenstoffatom der Carbonylgruppe gebunden ist. (Davon zu unterscheiden sind Thioncarbamate (Thionurethane) R-NH-(C=S)-O-R', bei denen im Vergleich zu Carbamaten das Sauerstoffatom der Carbonylgruppe durch Schwefel ersetzt ist. Als Oberbegriff für beide Substanzklassen wird häufig die Bezeichnung Thiocarbamate (Thiourethane) verwendet.) Siehe hierzu auch nachfolgendes Schema 1.

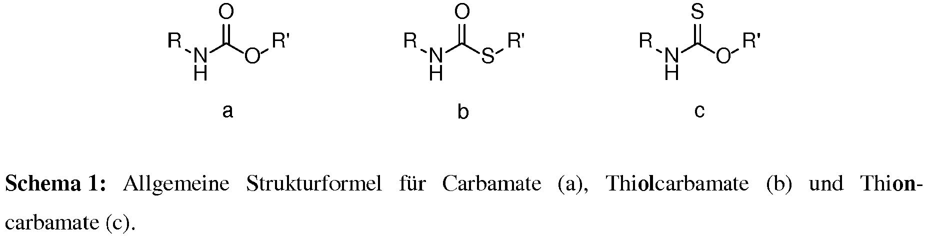

In der Terminologie dieser Erfindung umfassen die Begriffe Carbamat und Thiolcarbamat selbstverständlich auch Verbindungen mit mehr als einer, insbesondere mit zwei oder mehr, Carbamat- bzw. Thiolcarbamat-Gruppen. Solche weiteren Carbamat- bzw. Thiolcarbamat-Gruppen sind dann Bestandteil des Restes R der Strukturformeln aus Schema 1.

Die genannten *Katalysatoren* zeichnen sich dadurch aus, dass sie sowohl über eine Protonendonatorfunktion als auch über eine Protonenaktzeptorfunktion verfügen. In den erfindungsgemäßen Katalysatoren ist ein **abspaltbares Proton (H⁺)** an ein Heteroatom, das elektronegativer als Kohlenstoff ist, gebunden. Dieses Heteroatom ist entweder identisch mit Z (Z = O: **Z-H = ·O-H)** oder Bestandteil desselben (z. B. das Stickstoffatom im Fall Z = N(R⁶): **Z-H = C₆H₅(N·)H).** In den erfindungsgemäßen Katalysatoren ist ferner eine Protonenakzeptorfunktion enthalten, die entweder identisch mit X **(X = O)** oder ein Bestandteil desselben ist (z. B. das Stickstoffatom im Fall X = N(R¹): **X** = **C₆H₅-N:).** Erfindungsgemäß sind (X) und "Z" in (Z-H) über (Y) kovalent miteinander verbunden. Diese Verbindung kann durch eine Brücke aus 2 Kohlenstoffatomen oder durch eine Brücke aus 3, 5 oder 7 Kohlenstoffatomen mit alternierenden Einfach und Doppelbindungen realisiert sein. Die Verbindung kann auch durch ein einzelnes Kohlenstoffatom ((Y) = C(R²)) realisiert sein.

Ein Beispiel für einen *Katalysator vom Typ* (A) ist 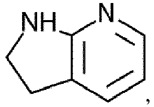 mit einem pK_{B}-Wert von 3,25, worin gilt: Z = N(R⁶), Y = C(R²) und X = N(R¹), wobei R¹ und R² einerseits zu einem Ring aus 6 Atomen, der das Heteroatom N (nämlich den "Pyridin"-Stickstoff) enthält, sowie R⁶ und R² andererseits zu einem Ring aus 5 Atomen, der das Heteroatom N (nämlich den das Proton tragenden Stickstoff) enthält, verbunden sind. In diesem Beispiel haben der Fünf- und der Sechsring zwei Kohlenstoffatome gemeinsam; dies ist von der Erfindung ausdrücklich umfasst (ist aber selbstverständlich nicht zwingend). Ferner ist in diesem Beispiel das C-Atom in C(R²) Bestandteil sowohl des Rings, der durch die Verbindung von R¹ und R² als auch des Rings, der durch die Verbindung von R⁶ und R² entsteht; auch dies ist von der Erfindung ausdrücklich umfasst. Im Rahmen dieser Erfindung werden die gemäß *Advanced Chemistry Development (ACD*/*Labs) Software V11.02* für 25 °C berechneten pK_{B}-Werte als maßgeblich angesehen. Diese sind für zahlreiche organische Verbindungen tabelliert und über die Substanzsuche der Chemical Abstracts Service SCIFINDER^{®} Datenbank unter *Substance Detail, Predicted Properties, Chemical,* zugänglich. Im Falle von Verbindungen des Typs (A) mit mehreren basischen Gruppen muss die erfindungsgemäße Forderung eines minimalen pK_{B}-Werts von 3,00 von der am stärksten basischen Gruppe erfüllt werden.

Ein weiteres Beispiel für einen Katalysator vom Typ (A) ist 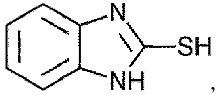 worin gilt Z = S, Y = C(R²), X = N(R¹), wobei R¹ mit R² zu einem aus 5 Atomen bestehenden Ring (dem Imidazolring) verbunden sind. In diesem Beispiel ist der aus der Verbindung von R¹ und R² resultierende Imidazolring mit einem Benzolring anelliert; dies ist von der Erfindung ausdrücklich umfasst.

Ein Beispiel für einen *Katalysator vom Typ (B)* ist 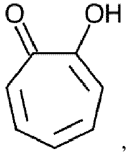 worin gilt: Z = O, Y = Brücke aus 2 Kohlenstoffatomen, die Bestandteil eines Ringsystems aus 7 Kohlenstoffatomen mit alternierenden Einfach- und Doppelbindungen sind, und X = O.

Ein Beispiel für einen *Katalysator vom Typ (C)* ist 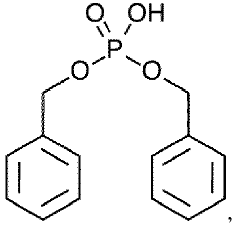 worin gilt: Z = O, Y = P(OR⁴)(OR⁵) und X =O, wobei ferner R⁴ und R⁵ araliphatische Reste mit jeweils 7 Kohlenstoffatomen sind.

Ohne auf eine Theorie festgelegt sein zu wollen, wird angenommen, dass das Vorhandensein von Protonendonatorfunktion und Protonenaktzeptorfunktion in demselben Molekül einen *Protonenshift* gestattet, der für die Carbamat- bzw. Thiolcarbamatspaltung wesentlich ist. Hierfür spricht auch, dass sich die genannten Katalysatoren formal in Strukturformeln darstellen lassen, in denen die Protonendonatorfunktion und Protonenaktzeptorfunktion über alternierende Atom-Atom-Einfachbindungen und Atom-Atom-Doppelbindungen verbunden sind.

Es folgt zunächst eine Kurzzusammenfassung verschiedener möglicher **Ausführungsformen der Erfindung:**
In einer **ersten Ausführungsform der Erfindung,** die mit allen anderen Ausführungsformen kombinierbar ist, wird die Umsetzung des Ausgangs-Carbamats bzw. -Thiolcarbamats in Lösung in Gegenwart eines organischen Lösungsmittels ausgewählt aus aprotischen polaren Lösungsmittel ohne Isocyanat-reaktive Gruppen durchgeführt.

In einer **zweiten Ausführungsform der Erfindung,** die eine besondere Ausgestaltung der ersten Ausführungsform ist, wird das organische Lösungsmittel ausgewählt aus Diphenylether, Sulfolan, zyklisches Propylencarbonat oder einer ionische Flüssigkeit (insbesondere 1 -Butyl-3 -methylimidazoliumhydrogensulfat, 1 -Butyl-3 -methylimidazoliummethansulfonat und/oder Trihexyltetradecylphosphonium-bis(2,4,4-trimethylpentyl)phosphinat).

In einer **dritten Ausführungsform der Erfindung,** die eine besondere Ausgestaltung der ersten und zweiten Ausführungsform ist, wird eine Konzentration des Ausgangs-Carbamats bzw. -Thiolcarbamats in der Lösung im Bereich von 5 Massen-% bis 95 Massen-%, bevorzugt im Bereich von 10 Massen-% bis 20 Massen-%, bezogen auf die Gesamtmasse der Lösung, eingestellt.

In einer **vierten Ausführungsform der Erfindung,** die mit allen anderen Ausführungsformen kombinierbar ist, wird ein molares Verhältnis von Ausgangs-Carbamat bzw. -Thiolcarbamat zu Katalysator von 1000 : 1 bis 1 : 1, bevorzugt von 100 : 1 bis 10 : 1, eingesetzt.

In einer **fünften Ausführungsform der Erfindung,** die mit allen anderen Ausführungsformen kombinierbar ist, wird die Umsetzung bei einer Temperatur im Bereich von 150 °C bis 280 °C und bei einem Druck im Bereich von 0,001 bar_{(abs.)} bis 2,00 bar_{(abs.)}, besonders bevorzugt bei einer Temperatur im Bereich von 160 °C bis 260 °C und bei einem Druck im Bereich von 0,001 bar_{(abs.)} bis 1,00 bar_{(abs.)}, ganz besonders bevorzugt bei einer Temperatur im Bereich von 180 °C bis 240 °C und bei einem Druck im Bereich von 0,001 bar_{(abs.)} bis 1,00 bar_{(abs.)} durchgeführt.

In einer **sechsten Ausführungsform der Erfindung,** die mit allen anderen Ausführungsformen kombinierbar ist, wird die Umsetzung kontinuierlich oder diskontinuierlich in einem Reaktor ausgewählt aus der Gruppe bestehend aus Rührkesseln, Rührkesselkaskaden, Destillationskolonnen und Rohrreaktoren durchgeführt.

In einer **siebten Ausführungsform der Erfindung,** die eine besondere Ausgestaltung der sechsten Ausführungsform ist, wird eine Verweilzeit des Reaktionsgemisches im Reaktor im Bereich von 0,5 h bis 10 h, bevorzugt im Bereich von 1,0 h bis 8,0 h, besonders bevorzugt im Bereich von 1,5 h bis 6,0 h, eingestellt.

In einer **achten Ausführungsform der Erfindung,** die mit allen anderen Ausführungsformen kombinierbar ist, wird das gebildete Isocyanat und/oder der gebildete Alkohol bzw. Thioalkohol kontinuierlich oder in Intervallen aus dem Reaktionsgemisch entfernt.

In einer **neunten Ausführungsform der Erfindung,** die eine besondere Ausgestaltung der achten Ausführungsform ist, wird *der gebildete Alkohol bzw. Thioalkohol* kontinuierlich oder in Intervallen aus dem Reaktionsgemisch entfernt, wobei das Entfernen des Alkohols bzw. Thioalkohols durch Durchleiten eines Stripgases (bevorzugt Stickstoff oder ein Edelgas wie insbesondere Helium oder Argon) und/oder destillativ bewirkt wird, optional unterstützt durch Anlegen eines gegenüber Umgebungsdruck verminderten Drucks.

In einer **zehnten Ausführungsform der Erfindung,** die eine weitere besondere Ausgestaltung der achten Ausführungsform ist, werden das gebildete Isocyanat *und* der gebildete Alkohol bzw. Thioalkohol kontinuierlich oder in Intervallen aus dem Reaktionsgemisch entfernt, wobei (i) entweder beide zusammen entfernt werden, gefolgt von einer Trennung des anfallenden gasförmigen Gemisches enthaltend das Isocyanat und den Alkohol bzw. Thioalkohol durch fraktionierende Kondensation, oder (ii) zuerst der Alkohol bzw. Thioalkohol und danach das Isocyanat aus dem Reaktionsgemisch entfernt wird. Die Abtrennung des gebildeten Isocyanats erfolgt vorteilhafterweise durch Destillation; im Falle der Variante (i) werden dann das gebildete Isocyanat und der gebildete Alkohol bzw. Thioalkohol gemeinsam aus dem Reaktionsgemisch abdestilliert, wobei die Destillation durch das Durchleiten eines Stripgases (bevorzugt Stickstoff oder ein Edelgas wie insbesondere Helium oder Argon) unterstützt werden kann. Im Fall der Variante (ii) kann die Abtrennung des Alkohols bzw. Thioalkohols durch Durchleiten eines Stripgases (bevorzugt Stickstoff oder ein Edelgas wie insbesondere Helium oder Argon) und/oder destillativ bewirkt werden; die Abtrennung des Isocyanats erfolgt vorzugsweise destillativ, wobei das Durchleiten eines Stripgases (bevorzugt Stickstoff oder ein Edelgas wie insbesondere Helium oder Argon) jedoch unterstützend eingesetzt werden kann.

In einer **elften Ausführungsform der Erfindung,** die eine besondere Ausgestaltung der zehnten Ausführungsform ist, wird das Reaktionsgemisch zur Entfernung des Alkohols bzw. Thioalkohols und des Isocyanats kontinuierlich in zwei hintereinandergeschalteten Destillationskolonnen destilliert.

In einer **zwölften Ausführungsform der Erfindung,** die mit allen anderen Ausführungsformen kombinierbar ist, ist das herzustellende Isocyanat
1,4-Butylendiisocyanat, 1,5-Pentandiisocyanat, 1,6-Hexamethylendiisocyanat oder deren Dimere, Trimere, Pentamere, Heptamere oder Nonamere oder Gemische derselben, Isophorondiisocyanat, 2,2,4- und/oder 2,4,4-Trimethylhexamethylendiisocyanat, die isomeren Bis(4,4'-isocyanatocyclohexyl)methane oder deren Mischungen in beliebigen Anteilen, 1,4-Cyclohexylendiisocyanat, Phenylisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat und/oder dessen höhere Homologe, 1,3- und/oder 1,4-Bis-(2-isocyanato-prop-2-yl)-benzol, 1,3-Bis-(isocyanatomethyl)benzol, oder ein Alkyl-2,6-diisocyanatohexanoat (Lysindiisocyanat) mit Alkylgruppen aus 1 Kohlenstoffatom bis 6 Kohlenstoffatomen,
und als Carbamat bzw. Thiolcarbamat wird das zu dem herzustellenden Isocyanant korrespondierende
   Methyl-, Ethyl-, *n*-Propyl-, *iso*-Propyl-, *n*-Butyl-, *iso*-Butyl-, *tert*-Butyl-*,* Cyclohexyl- oder Phenyl-Carbamat bzw. -Thiolcarbamat
      oder
   substituierte Methyl-, Ethyl-, *n*-Propyl-, *iso*-Propyl-, *n*-Butyl-, *iso*-Butyl-, *tert*-Butyl*-,* Cyclohexyl- oder Phenyl-Carbamat bzw. -Thiolcarbamat
eingesetzt.

In einer **dreizehnten Ausführungsform der Erfindung,** die mit allen anderen Ausführungsformen kombinierbar ist, wird neben dem Katalysator (X)(Y)(Z)H kein weiterer Katalysator eingesetzt.

In einer **vierzehnten Ausführungsform der Erfindung,** die mit allen anderen Ausführungsformen kombinierbar ist, ist der Katalysator (X)(Y)(Z-H) ausgewählt ist aus der Gruppe bestehend aus
2-Hydroxy-2,4,6-cycloheptatrien-1-on (Tropolon) **(a);** ; N,N'-Diphenylformamidin **(b);** *N-*(2,6-Dimethylphenyl)-5,6-dihydro-4*H*-1,3-thiazin-2-amin (Xylazin) **(c);** 2,3-Dihydro-7-azaindol **(d);** protoniertes N-Methyl-1,5,7-triazabicyclo[4.4.0]dec-5-en **(e);** protoniertes 1,8-Diazabicyclo[5.4.0]undec-7-en **(f);** O-Methyl-N,N'-diisopropylisoharnstoff **(g);** 2-Mercaptopyridin **(h);** 1,3,4-Thiadiazol-2,5-dithiol **(i);** Mercaptobenzimidazol **(j);** den Konstitutionsisomeren des Benzoldisulfonsäure-Monoanions **(k);** den Konstitutionsisomeren der Benzoldisulfonsäure **(l);** (R)-(-)-1,1'-Binaphthyl-2,2'-hydrogenphosphat **(m)**; Dibenzylhydrogenphosphat **(n);** 2,6-Naphtalindisulfonsäure-Monoanion **(o);** Alkalimetallhydrogensulfat **(p),** 2-Aminoethan-1-Sulfonsäure (Taurin) **(q)** und Mischungen **(r)** der vorgenannten Verbindungen.

In einer **fünfzehnten Ausführungsform** der Erfindung, die insbesondere (aber nicht nur) mit der vierzehnten Ausführungsform kombiniert werden kann, werden als Katalysatoren solche des Typs (A) eingesetzt, vorzugsweise ausschließlich solche des Typs (A).

In einer **siebzehnten Ausführungsform** der Erfindung, die insbesondere (aber nicht nur) mit der vierzehnten Ausführungsform kombiniert werden kann, werden als Katalysatoren solche des Typs (B) eingesetzt, vorzugsweise ausschließlich solche des Typs (B).

In einer **achtzehnten Ausführungsform** der Erfindung, die insbesondere (aber nicht nur) mit der vierzehnten Ausführungsform kombiniert werden kann, werden als Katalysatoren solche des Typs (C) eingesetzt, vorzugsweise ausschließlich solche des Typs (C).

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Erfindungsgemäß einsetzbare *Ausgangs-Carbamate* können über verschiedene, an sich bekannte Wege erhalten werden. Beispielhaft seien die Umesterung von N-Aryl-Harnstoffderivaten mit Alkoholen (beschrieben beispielsweise in J. Wang et al., Applied Catalysis A: General, 2004, 261, 191-197), die reduktive Carbonylierung von Nitroaromaten mit Kohlenstoffmonoxid und Alkoholen (beschrieben beispielsweise in M.vGasperini et al., Adv. Syn. Cat., 2005, 347, 105-120), die oxidative Carbonylierung von Aminen mit Kohlenstoffmonoxid und Sauerstoff (beschrieben beispielsweise in S. Fukuoka, M. Chono, M. Kohno, J. Chem. Soc., Chem. Commun., 1984, 399) und die Umsetzung primärer Amine mit organischen Carbonaten (beschrieben beispielsweise in US 8,871,965 B2) genannt.

Erfindungsgemäß bevorzugt ist die Umsetzung primärer Amine mit organischen Carbonaten, welche gemäß

R-NH₂ + R'₂CO₃ -> R-NH-(C=O)-O-R' + R'-OH

abläuft.

Bevorzugt werden Verfahren eingesetzt, wie sie z. B. in WO 2014/187756 A1 und den dort zitierten Literaturstellen beschrieben werden. Diese Reaktionen werden z. B. durch Zinkcluster, Zinksalze oder Lewissäuren katalysiert.

Erfindungsgemäß einsetzbare *Ausgangs-Thiolcarbamate* können über verschiedene, an sich bekannte Wege erhalten werden. Beispiele aus der Fachliteratur sind etwa in Tetrahedron 50 (1994) 5669-5680, Tetrahedron 59 (2003) 1327-1331, Tetrahedron 60 (2004) 2869-2873, Tetrahedron 61 (2005) 7153-7175, J. Org. Chem. 68 (2003) 3733-3735, J. Org. Chem. 70 (2005) 2551-2554 und US 4,486,449 offenbart. Erfindungsgemäß einsetzbare Ausgangs-Thiolcarbamate werden besonders bevorzugt erhalten durch die Carbonylierung von Aminen mit Kohlenstoffmonoxid, Schwefel und Alkylhalogeniden.

Bevorzugt wird die Umsetzung des Ausgangs-Carbamats bzw. -Thiolcarbamats in Lösung durchgeführt. Als Lösungsmittel geeignet sind insbesondere aprotische polare Lösungsmittel ohne Isocyanat-reaktive Gruppen. Bevorzugt sind Diphenylether, Sulfolan, zyklisches Propylencarbonat oder ionische Flüssigkeiten (insbesondere 1-Butyl-3-methylimidazoliumhydrogensulfat, 1-Butyl-3-methylimidazoliummethansulfonat und/oder Trihexyltetradecylphosphonium-bis(2,4,4-trimethylpentyl)phosphinat). Die Konzentration des Ausgangs-Carbamats bzw. -Thiolcarbamats in der Lösung liegt bevorzugt im Bereich von 5 Massen-% bis 95 Massen-%, besonders bevorzugt im Bereich von 10 Massen-% bis 20 Massen-%, bezogen auf die Gesamtmasse der Lösung. Bevorzugt wird ein molares Verhältnis von Ausgangs-Carbamat bzw. -Thiolcarbamat zu Katalysator von 1000 : 1 bis 1 : 1, bevorzugt von 100 : 1 bis 10 : 1, eingesetzt. Eine lösungsmittelfreie Synthese ist aber ebenfalls möglich.

Das erfindungsgemäße Verfahren wird bevorzugt bei einer Temperatur im Bereich von 150 °C bis 280 °C und bei einem Druck im Bereich von 0,001 bar_{(abs.)} bis 2,00 bar_{(abs.)}, besonders bevorzugt bei einer Temperatur im Bereich von 160 °C bis 260 °C und bei einem Druck im Bereich von 0,001 bar_{(abs.)} bis 1,00 bar_{(abs.)}, ganz besonders bevorzugt bei einer Temperatur im Bereich von 180 °C bis 240 °C und bei einem Druck im Bereich von 0,001 bar_{(abs.)} bis 1,00 bar_{(abs.)} durchgeführt. Das erfindungsgemäße Verfahren kann sowohl kontinuierlich als auch diskontinuierlich ("batchweise") betrieben werden. Geeignete Reaktoren zur Durchführung des Verfahrens sind insbesondere Rührkessel oder Rührkesselkaskaden, Destillationskolonnen (Reaktivdestillation) oder Rohrreaktoren. Die Verweilzeit des Reaktionsgemisches im eingesetzten Reaktor beträgt dabei bevorzugt von 0,5 h bis 10 h, besonders bevorzugt von 1,0 h bis 8,0 h, ganz besonders bevorzugt von 1,5 h bis 6,0 h.

Insbesondere bevorzugt ist es, das gebildete Isocyanat und/oder den gebildeten Alkohol bzw. Thioalkohol aus dem Reaktionsgemisch kontinuierlich oder in Intervallen zu entfernen. Dies kann durch Durchleiten eines Stripgases (bevorzugt Stickstoff oder ein Edelgas wie insbesondere Helium oder Argon) und/oder destillativ bewirkt werden, optional unterstützt durch Anlegen eines gegenüber Umgebungsdruck verminderten Drucks. Dabei können insbesondere im Fall der Destillation die Bedingungen so gewählt werden, dass beide gemeinsam abdestillieren. In diesem Fall ist es bevorzugt, das gasförmig anfallende abdestillierte Gemisch enthaltend das herzustellende Isocyanat und den Alkohol bzw. Thioalkohol (sowie ggf. leicht siedende Nebenkomponenten) durch fraktionierende Kondensation in zwei oder mehr Schritten zu trennen und auf diese Weise das Isocyanat und den Alkohol bzw. Thioalkohol zu gewinnen.

Es ist auch denkbar, nur die niedriger siedende Komponente (im Allgemeinen der Alkohol bzw. Thioalkohol) aus dem Reaktionsgemisch zu entfernen *(weniger bevorzugt),* oder zuerst die niedriger siedende und dann die höher siedende *(stärker bevorzugt).* Bei kontinuierlicher Fahrweise kann die letztgenannte Variante am einfachsten durch Hintereinanderschaltung von zwei Destillationskolonnen verwirklicht werden. Im Falle von Polycarbamaten bzw. -Thiolcarbamaten ist darauf zu achten, dass das korrespondierende Polyisocyanat (meist ein Diisocyanat) erst dann abdestilliert wird, wenn alle Carbamat- bzw. Thiolcarbamatgruppen der Ausgangsverbindung in Isocyanatgruppen überführt sind (es sei denn, das Zielprodukt wäre ein gemischtes (Thiolo-)Carbamat-Isocyanat, was jedoch im Allgemeinen nicht der Fall sein wird).

Geeignete *Carbamate* sind insbesondere solche Carbamate, die sich retrosynthetisch zurückführen lassen auf die Reaktion von
(1) primären, sekundären oder tertiären (gegebenenfalls substituierten) aliphatischen Monoalkoholen R'-OH wie
   Methanol, Ethanol, n-Propanol, iso-Propanol, n-Butanol, iso-Butanol, tert-Butanol, deren höheren Homologen, Cyclohexanol, wobei die primären Monoalkohole bevorzugt sind,
   oder (gegebenenfalls substituiertes) Phenol
   mit
(2) Isocyanaten R-NCO wie
   1,4-Butylendiisocyanat, 1,5-Pentandiisocyanat (PDI), 1,6-Hexamethylendiisocyanat (HDI) bzw. deren Dimere, Trimere, Pentamere, Heptamere oder Nonamere oder Gemische derselben, Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen in beliebigen Anteilen, 1,4-Cyclohexylendiisocyanat, Phenylisocyanat, 1,4-Phenylendiisocyanat, 2,4- und/oder 2,6-Toluylendiisocyanat (TDI), 1,5-Naphthylendiisocyanat, 2,2'- und/oder 2,4'- und/oder 4,4'-Diphenylmethandiisocyanat (MDI) und/oder höhere Homologe (polymeres MDI), 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit Alkylgruppen aus 1 Kohlenstoffatom bis 6 Kohlenstoffatomen.

Geeignete *Thiolcarbamate* sind insbesondere Verbindungen, die retrosynthetisch auf die Reaktion der zu den zuvor genannten Monoalkoholen (1) korrespondierenden Monothioalkoholen R`-SH (1' - Ersatz von O durch S) mit den genannten Isocyanaten (2) zuzurückzuführen sind. Besonders bevorzugt sind dabei Thiomethanol, Thioethanol, Thioisopropanol und (gegebenenfalls substituiertes) Thiophenol.

In den Formeln R-NH-(C=O)-O-R' und R-NH-(C=O)-S-R' entspricht daher R dem Rest des Isocyanats (also z. B. C₆H₅ im Fall von Phenylisocyanat; im Fall von Isocyanaten mit mehr als einer Isocyanatgruppe weist auch das korrespondierende Carbamat die entsprechende Anzahl an Carbamatfunktionen R`-O-(CO)-NH- auf) und R' dem Rest des Monoalkohols (z. B. CH₃ im Fall von Methanol).

Im Falle von substituierten Alkyl-Carbamaten bzw. Alkyl-Thiolcarbamaten (R-NH-(CO)-O-R') trägt der aliphatische Rest R' Substituenten, die unabhängig voneinander ausgewählt sind aus der Gruppe bestehend aus CN, NO₂, F, Cl, Br, I, OR", wobei R" für eine Alkylgruppe, insbesondere für CH₃, C₂H₆, *n*-C₃H₇, *i*-C₃H₇, *n*-C₄H₉, *i*-C₄H₉, *t*-C₄H₉, *n*-C₅H₁₁ oder *n*-C₆H₁₃, steht und R die gleiche Bedeutung wie zuvor hat. Dabei ist mindestens ein Wasserstoffatom des aliphatischen Restes R' durch einen der genannten Substituenten ersetzt.

Im Falle von substituierten Phenyl-Carbamaten (R-NH-(CO)-O-C₆Hₘ₋₅Aₘ) bzw. Phenyl-Thiolcarbamaten (R-NH)-(CO)-S-C₆Hₘ₋₅Aₘ) trägt der aromatische Sechsring bis zu m Substituenten A, die unabhängig voneinander ausgewählt sind aus der Gruppe bestehend aus CN, NO₂, F, Cl, Br, I, OH, COOH, COCl, COOR", OR", CH₃, C₂H₆, *n*-C₃H₇, *iso*-C₃H₇, wobei m für eine natürliche Zahl im Bereich von 0 bis 5, bevorzugt von 0 bis 4, besonders bevorzugt von 0 bis 1, und R" für eine Alkylgruppe, insbesondere für CH₃, C₂H₆, *n*-C₃H₇, *iso*-C₃H₇, *n*-C₄H₉, *iso*-C₄H₉, *tert-*C₄H₉*, n*-C₅H₁₁ oder *n*-C₆H₁₃, steht und R die gleiche Bedeutung wie zuvor hat.

In allen Ausführungsformen der Erfindung ist es bevorzugt, neben dem Katalysator (X)(Y)(Z)H *keine anderen Katalysatoren* einzusetzen. Das erfindungsgemäße Verfahren ermöglicht also - sieht man bei salzartigen Katalysatoren von einem gegebenfalls eingesetzten metallischen Kation ab - den völligen Verzicht auf metallhaltige Katalysatoren, was den Vorteil hat, dass der Prozess insgesamt umweltfreundlicher wird und mit einem geringeren Aufarbeitungsaufwand des Rohproduktes auskommt. Dies bedeutet, dass sowohl eine weniger starke Aufreinigung des Produktes erforderlich ist, da Katalysatorrückstände weniger toxisch sind und somit im Produkt verbleiben können, als auch, dass entstehende Abfälle einfacher entsorgt werden können. Des Weiteren ist der im Stand der Technik übliche Einsatz von Metallen, speziell im Bereich der edleren Metalle, oft mit hohen Kosten verbunden.

Neben den Katalysatorstrukturen (X)(Y)(Z)H selbst können auch deren unter Reaktionsbedingungen gebildete Folgeprodukte katalytisch aktiv sein.

Der *Katalysator (X)(Y)(Z)H* ist bevorzugt ausgewählt aus der Gruppe bestehend aus (siehe auch das folgende Schema 2) 2-Hydroxy-2,4,6-cycloheptatrien-1-on (Tropolon) **(a);** N,N'-Diphenylformamidin (**b; pK_{B} = 6,30**); *N*-(2,6-Dimethylphenyl)-5,6-dihydro-4*H*-1,3-thiazin-2-amin (Xylazin) **(c; pK_{B} = 6,33);** 2,3-Dihydro-7-azaindol **(d; pK_{B} = 3,25);** protoniertes N-Methyl-1,5,7-triazabicyclo[4.4.0]dec-5-en **(e);** protoniertes 1,8-Diazabicyclo[5.4.0]undec-7-en **(f);** O-Methyl-N,N'-diisopropylisoharnstoff (**g; pK_{B} = 4,15**); 2-Mercaptopyridin **(h; pK_{B}** = **4,24);** 1,3,4-Thiadiazol-2,5-dithiol (i; **pK_{B} = 8,34);** Mercaptobenzimidazol **(j);** den Konstitutionsisomeren des Benzoldisulfonsäure-Monoanions (= Sulfobenzolsulfonat) **(k);** den Konstitutionsisomeren der Benzoldisulfonsäure **(l);** (R)-(-)-1,1'-Binaphthyl-2,2'-hydrogenphosphat **(m);** Dibenzylhydrogenphosphat **(n);** 2,6-Naphtalindisulfonsäure-Monoanion **(o);** Alkalimetallhydrogensulfat **(p),** 2-Aminoethan-1-Sulfonsäure (Taurin) **(q)** und Mischungen **(r)** der vorgenannten Verbindungen. Für die protonierten Verbindungen (Salze) **(e)** und **(f)** sind nach diesseitigem Kenntnisstand keine pK_{B}-Werte in der Literatur dokumentiert; jedoch ist für den Fachmann augenscheinlich, dass diese Verbindungen dem erfindungsgemäßen Kriterium (pK_{B} ≥ 3,00) genügen, da beide bereits protoniert sind und somit das Bestreben, ein weiteres Proton aufzunehmen, äußerst gering (und damit der pK_{B}-Wert im Vergleich zur unprotonierten Spezies höher) ist.

| | | | |
|---|---|---|---|
| 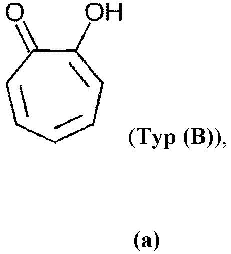 | | 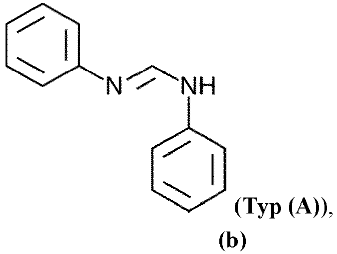 | |
| 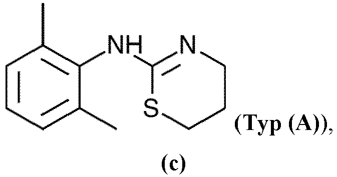 | 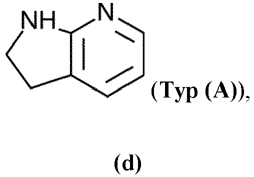 | | 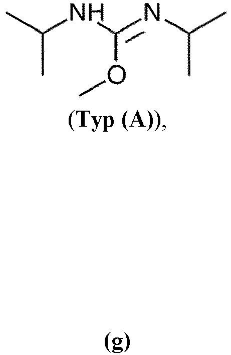 |
| 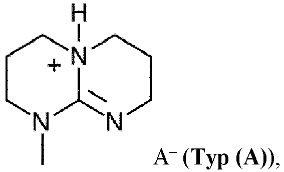 wobei A⁻ für ein nicht nucleophiles Anion, insbesondere ausgewählt aus der Gruppe bestehend aus Tosylat, Bis-(Trifluoromethyl-sulfonyl)imid, *tert*.-Butoxid, Diisopropylamid, Hexafluorophosphat, Tetrafluoroborat, Perchlorat und Tetrakis[3,5-bis-(trifluoromethyl)phenyl] borat ("BARF"), steht, | 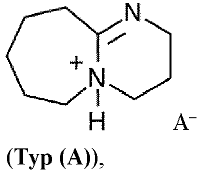 wobei A⁻ die bereits genannte Bedeutung hat, | | |
| (e) | (f) | | |
| 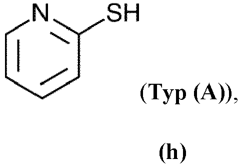 | 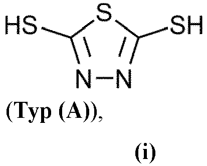 | | 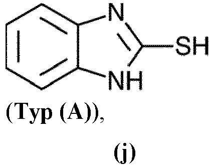 |

### Schema 2: Strukturen bevorzugter Katalysatoren (Fortsetzung auf nächster Seite).

| | | |
|---|---|---|
| 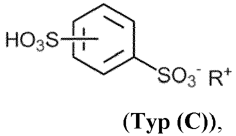 wobei R⁽⁺⁾ für ein Kation, bevorzugt ein Alkalimetallkation, Imidazoliumkation, Pyridiniumkation, Pyrrolidiniumkation, Phosphoniumkation, Sulfoniumkation, NH₄⁺ oder für ein mono-, di-, tri- oder tetra-substituiertes organisches Ammoniumkation, dessen organische Substituenten unabhängig voneinander ausgewählt sind aus der Gruppe bestehend aus Methyl, Ethyl, Propyl, Butyl, Pentyl, Hexyl, Phenyl und Cyclohexyl, steht, wobei besonders bevorzugt R⁽⁺⁾ für ein Alkalimetallkation oder NH₄⁺, ganz besonders bevorzugt für ein Alkalimetallkation ausgewählt aus Li⁺, Na⁺ oder K⁺, steht, | 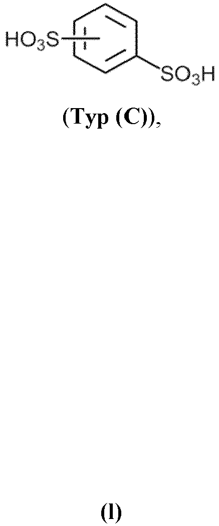 | 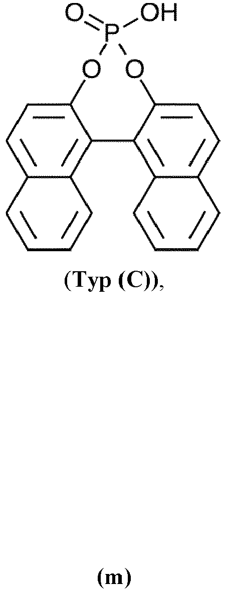 |
| (k) | | |
| 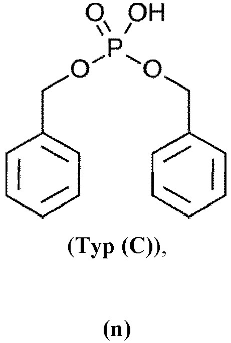 | 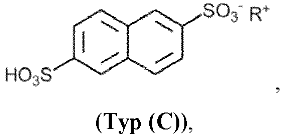 wobei R⁽⁺⁾ die bereits genannte Bedeutung hat, | 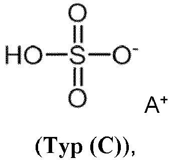 wobei A⁺ für ein Alkalimetallkation, insbesondere für Na⁺, steht, |
| | **(o)** | **(p)** |
| 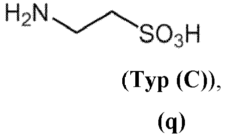 | und Mischungen der vorgenannten Verbindungen. | |
| | **(r)** | |

### Schema 2: Strukturen bevorzugter Katalysatoren (fortgesetzt).

In einer Ausführungsform der Erfindung werden Katalysatoren vom Typ (A) eingesetzt, insbesondere ausschließlich solche vom Typ (A). Dabei sind die in Schema 2 genannten Katalysatoren des Typs (A) besonders bevorzugt.

In einer anderen Ausführungsform der Erfindung werden Katalysatoren vom Typ (B) eingesetzt, insbesondere ausschließlich solche vom Typ (B). Dabei sind die in Schema 2 genannten Katalysatoren des Typs (B) besonders bevorzugt.

In einer Ausführungsform der Erfindung werden Katalysatoren aus der Typ (C) eingesetzt, insbesondere ausschließlich solche vom Typ (C). Dabei sind die in Schema 2 genannten Katalysatoren der Typs (C) besonders bevorzugt.

Von den in Schema 2 genannten Katalysatoren sind Alkalimetallhydrogensulfat (insbesondere Natriumhydrogensulfat) **(p)),** und 1,3-Benzoldisulfonsäure-Monoanion (3-Sulfobenzolsulfonat) **(k)** (beide vom Typ (C)) ganz besonders bevorzugt.

Erfindungsgemäß einsetzbare Katalysatoren sind kommerziell verfügbar oder zumindest nach bekannten Methoden erhältlich. So sind etwa Katalysatoren in protonierter Form **((f)** und **(g))** durch Umsetzung der neutralen Form mit der Säure A-H, beispielsweise Trifluoromethansulfonsäure, erhältlich. Die Verbindungen **(l)** und **(p)** lassen sich beispielsweise durch Umsetzung der korrespondierenden Dianionen, bspw. der Di-Natriumsalze, mit einer Säure wie insbesondere Schwefelsäure oder Trifluoromethansulfonsäure erhalten.

Nachstehend wird die Erfindung anhand von Beispielen noch näher erläutert.

### Beispiele:

Die Versuche wurden in Standard-Labor-Apparaturen durchgeführt. Die Inertisierung der Reaktionsgefäße erfolgte mit Argon. Phenanthren wurde als interner Standard für quantitative HPLC-Analytik verwendet.

### Beispiel 1: Umsetzung von Methyl-N-Phenylcarbamat zu Phenylisocyanat durch thermische Spaltung bei 200 °C in Diphenylether (Vergleichsbeispiel ohne Katalysator)

In einem inertisierten Mehrhalskolben wurden 0,61 g (3,42 mmol) Phenanthren in 29,34 g (172,38 mmol) Diphenylether gelöst. Das Reaktionsgemisch wurde auf 215 °C erwärmt. In einem inertisierten Schlenkrohr wurden 4,97 g (32,88 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 20 % mit einer Selektivität von 91 %.

### Beispiel 2: Umsetzung Methyl-N-Phenylcarbamat zu Phenylisocyanat durch thermische Spaltung bei 200 °C in Sulfolan (Vergleichsbeispiel ohne Katalysator)

In einem inertisierten Mehrhalskolben wurden 0,75 g (4,21 mmol) Phenanthren in 25,14 g (209,20 mmol) Sulfolan gelöst. Das Reaktionsgemisch wurde auf 217 °C erwärmt. In einem inertisierten Schlenkrohr wurden 6,35 g (42,01 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 17 % mit einer Selektivität von 87 %.

### Beispiel 3: Umsetzung von Methyl-N-Phenylcarbamat zu Phenylisocyanat durch Spaltung bei 60 °C in Gegenwart Natrium-3-sulfobenzolsulfonat bei einem molaren Verhältnis von Carbamat zu Katalysator von 19,5: 1 (Vergleichsbeispiel bei für die gewählte Katalysatorkonzentration zu geringer Temperatur)

In einem inertisierten Mehrhalskolben wurden 0,59 g (3,31 mmol) Phenanthren sowie 0,65 g (1,71 mmol) Natrium-3-sulfobenzolsulfonat in 30,01 g (176,31 mmol) Diphenylether suspendiert. Zu diesem Reaktiongemisch wurden 5,03 g (33,27 mmol) Methyl-N-Phenylcarbamat hinzugefügt und auf 60 °C erwärmt. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Es konnte keine Isocyanatbildung beobachtet werden.

### Beispiel 4: Umsetzung von Methyl-N-Phenylcarbamat zu Phenylisocyanat durch Spaltung bei 200 °C in Gegenwart von Natrium-3-sulfobenzolsulfonat (Katalysator des Typs (C)) bei einem molaren Verhältnis von Carbamat zu Katalysator von 21,3 : 1

In einem inertisierten Mehrhalskolben wurden 0,60 g (3,37 mmol) Phenanthren sowie 0,59 g (1,55 mmol) Natrium-3-sulfobenzolsulfonat in 30,01 g (176,31 mmol) Diphenylether suspendiert. Das Reaktionsgemisch wurde auf 215 °C erwärmt. In einem inertisierten Schlenkrohr wurden 4,99 g (33,01 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 32 % mit einer Selektivität von 74 %.

### Beispiel 5: Umsetzung Methyl-N-Phenylcarbamat zu Phenylisocyanat durch Spaltung bei 240 °C in Gegenwart Natrium-3-sulfobenzolsulfonat (Katalysator des Typs (C)) bei einem molaren Verhältnis von Carbamat zu Katalysator von 19,5 : 1

In einem inertisierten Mehrhalskolben wurden 0,60 g (3,37 mmol) Phenanthren sowie 0,66 g (1,74 mmol) Natrium-3-sulfobenzolsulfonat in 30,11 g (176,90 mmol) Diphenylether suspendiert. Das Reaktionsgemisch wurde auf 261 °C erwärmt. In einem inertisierten Schlenkrohr wurden 5,13 g (33,94 mmol) Methyl-N-Phenylcarbamat auf 178 °C erwärmt. Durch das vollständige Überführen des Methyl-N-Phenylcarbamat in das Reaktionsgemisch resultierte ein Gemisch mit einer Temperatur von 240 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 68 % mit einer Selektivität von 69 %.

### Beispiel 6: Umsetzung Methyl-N-Phenylcarbamat zu Phenylisocyanat durch Spaltung bei 200 °C in Gegenwart Natrium-3-sulfobenzolsulfonat (Katalysator des Typs (C)) bei einem molaren Verhältnis von Carbamat zu Katalysator von 1,01 : 1

In einem inertisierten Mehrhalskolben wurden 0,50 g (2,81 mmol) Phenanthren sowie 9,99 g (26,26 mmol) Natrium-3-sulfobenzolsulfonat in 25,02 g (146,99 mmol) Diphenylether suspendiert. Das Reaktionsgemisch wurde auf 215 °C erwärmt. In einem inertisierten Schlenkrohr wurden 4,01 g (26,53 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 69 % mit einer Selektivität von 81 %.

### Beispiel 7: Umsetzung Methyl-N-Phenylcarbamat zu Phenylisocyanat durch Spaltung bei 200 °C in Gegenwart N,N'-Diphenylformamidin (Katalysator des Typs (A)) bei einem molaren Verhältnis von Carbamat zu Katalysator von 19,1 : 1

In einem inertisierten Mehrhalskolben wurden 0,81 g (4,54 mmol) Phenanthren sowie 0,44 g (2,24 mmol) N,N'-Diphenylformamidin in 25,35 g (148,93 mmol) Diphenylether gelöst. Das Reaktionsgemisch wurde auf 216 °C erwärmt. In einem inertisierten Schlenkrohr wurden 6,48 g (42,87 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temeperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 32% mit einer Selektivität von 84%.

### Beispiel 8: Umsetzung Dimethyl-2,4-Toluol-dicarbamat zu 2,4-Toluol-diisocvanat durch Spaltung bei 200 °C in Gegenwart Natrium-3-sulfobenzolsulfonat (Katalysator des Typs (C)) bei einem molaren Verhältnis von Carbamat zu Katalysator von 10,1 : 1

In einem inertisierten Mehrhalskolben wurden 0,62 g (3,48 mmol) Phenanthren sowie 1,27 g (3,34 mmol) Natrium-3-sulfobenzolsulfonat in 30,42 g (178,72 mmol) Diphenylether suspendiert. Dem Reaktionsgemisch wurden 8,02 g (33,66 mmol) Dimethyl-2,4-Toluol-dicarbamat zugefügt und auf 200 °C erwärmt. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an 2,4-Toluol-diisocyanat betrug 50 % mit einer Selektivität von 86 %.

### Beispiel 9: Umsetzung Methyl-N-Octylcarbamat zu n-Octylisocyanat durch Spaltung bei 200 °C in Gegenwart Natrium-3-sulfobenzolsulfonat (Katalysator des Typs (C)) bei einem molaren Verhältnis von Carbamat zu Katalysator von 19,5 : 1

In einem inertisierten Mehrhalskolben wurden 0,49 g (2,75 mmol) Phenanthren sowie 0,52 g (1,37 mmol) Natrium-3-sulfobenzolsulfonat in 25,30 g (148,64 mmol) Diphenylether suspendiert. Das Reaktionsgemisch wurde auf 215 °C erwärmt. In einem inertisierten Schlenkrohr wurden 5,00 g (26,70 mmol) Methyl-N-Octylcarbamat auf 150 °C erwärmt. Durch das vollständige Überführen des Methyl-N-Octylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an n-Octylisocyanat betrug 31 % mit einer Selektivität von 84 %.

### Beispiel 10: Umsetzung Methyl-N-Phenylcarbamat zu Phenylisocyanat durch Spaltung bei 200 °C in Gegenwart von Tropolon (Katalysator des Typs (B)) bei einem molaren Verhältnis von Carbamat zu Katalysator von 18,0 : 1

In einem inertisierten Mehrhalskolben wurden 0,60 g (3,37 mmol) Phenanthren sowie 0,23 g (1,88 mmol) Tropolon in 25,78 g (151,46 mmol) Diphenylether gelöst. Das Reaktionsgemisch wurde auf 215 °C erwärmt. In einem inertisierten Schlenkrohr wurden 5,11 g (33,80 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temeperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 19 % mit einer Selektivität von 86 %.

### Beispiel 11: Umsetzung von Methyl-N-Phenylcarbamat zu Phenylisocyanat durch Spaltung bei 200 °C in Gegenwart von Triazabicyclodecen bei einem molaren Verhältnis von Carbamat zu Katalysator von 18,5 : 1 (Vergleichsbeispiel mit einem Katalysator mit einem zu kleinem pK_{B}-Wert

In einem inertisierten Mehrhalskolben wurden 0,75 g (4,21 mmol) Phenanthren sowie 0,31 g (2,23 mmol) 3,4,6,7,8,9-Hexahydro-2H-pyrimido[1,2-a]pyrimidine (Triazabicyclodecen, TBD, pK_{B} = -0,47) in 30,02 g (176,37 mmol) Diphenylether gelöst. Das Reaktionsgemisch wurde auf 215 °C erwärmt. In einem inertisierten Schlenkrohr wurden 6,25 g (41,34 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 2 % mit einer Selektivität von 2 %.

### Beispiel 12: Umsetzung Methyl-N-Phenylcarbamat zu Phenylisocyanat durch Spaltung bei 200 °C in Gegenwart von Natriumhydrogensulfat (Katalysator des Typs (C)) bei einem molaren Verhältnis von Carbamat zu Katalysator von 18,3 : 1

In einem inertisierten Mehrhalskolben wurden 0,63 g (3,53 mmol) Phenanthren sowie 0,22 g (1,83 mmol) Natriumhydrogensulfat in 30,64 g (180,16 mmol) Diphenylether gelöst. Das Reaktionsgemisch wurde auf 215 °C erwärmt. In einem inertisierten Schlenkrohr wurden 5,07 g (33,56 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temeperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 42 % mit einer Selektivität von 90 %.

### Beispiel 13: Umsetzung Methyl-N-Phenylcarbamat zu Phenylisocyanat durch Spaltung bei 200 °C in Gegenwart von Natriumsulfat bei einem molaren Verhältnis von Carbamat zu Katalysator von 19,3 : 1 (Vergleichsbeispiel zu Beispiel 12)

In einem inertisierten Mehrhalskolben wurden 0,59 g (3,31 mmol) Phenanthren sowie 0,25 g (1,76 mmol) Natriumsulfat in 30,17 g (177,25 mmol) Diphenylether gelöst. Das Reaktionsgemisch wurde auf 216 °C erwärmt. In einem inertisierten Schlenkrohr wurden 5,13 g (33,96 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 17 % mit einer Selektivität von 92 %.

### Beispiel 14: Umsetzung Methyl-N-Phenylcarbamat zu Phenylisocyanat durch Spaltung bei 200 °C in Gegenwart von Schwefelsäure bei einem molaren Verhältnis von Carbamat zu Katalysator von 19,1 : 1 (Vergleichsbeispiel zu Beispiel 12)

In einem inertisierten Mehrhalskolben wurden 0,62 g (3,48 mmol) Phenanthren sowie 0,17 g (1,73 mmol) Schwefelsäure in 30,44 g (178,83 mmol) Diphenylether gelöst. Das Reaktionsgemisch wurde auf 216 °C erwärmt. In einem inertisierten Schlenkrohr wurden 4,99 g (33,03 mmol) Methyl-N-Phenylcarbamat auf 150 °C erwärmt. Durch das vollständige Überführen des Methyl-N-Phenylcarbamat in das Reaktionsgemisch resultierte ein Gemisch mit einer Temeperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels ¹H-NMR-Spektroskopie verfolgt.

Die Ausbeute an Phenylisocyanat betrug 17 % mit einer Selektivität von 89 %.

### Beispiel 15: Umsetzung von 4-Methoxyphenyl-N-Phenylcarbamat zu Phenylisocyanat durch Spaltung bei 200 °C in Gegenwart von Natriumhydrogensulfat (Katalysator des Typs (C)) bei einem molaren Verhältnis von Carbamat zu Katalysator von 15,9 : 1

In einem inertisierten Mehrhalskolben wurden 0,62 g (3,48 mmol) Phenanthren sowie 0,25 g (2,08 mmol) Natriumhydrogensulfat in 29,95 g (175,96 mmol) Diphenylether gelöst. Das Reaktionsgemisch wurde auf 215 °C erwärmt. In einem inertisierten Schlenkrohr wurden 8,05 g (33,09 mmol) 4-Methoxyphenyl-N-Phenylcarbamat auf 150 °C erwärmt. Durch das vollständige Überführen des 4-Methoxyphenyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels HPLC Chromatographie verfolgt.

Die Ausbeute an Phenylisocyanat betrug 50 % mit einer Selektivität von 71 %.

### Beispiel 16: Umsetzung von 4-Methoxyphenyl-N-Phenylcarbamat zu Phenylisocyanat durch thermische Spaltung bei 200 °C in Diphenylether (Vergleichsbeispiel zu Beispiel 15)

In einem inertisierten Mehrhalskolben wurden 0,61 g (3,42 mmol) Phenanthren in 30,10 g (176,84 mmol) Diphenylether gelöst. Das Reaktionsgemisch wurde auf 215 °C erwärmt. In einem inertisierten Schlenkrohr wurden 8,03 g (33,01 mmol) 4-Methoxyphenyl-N-Phenylcarbamat auf 150 °C erwärmt. Durch das vollständige Überführen des 4-Methoxyphenyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels HPLC Chromatographie verfolgt.

Die Ausbeute an Phenylisocyanat betrug 27 % mit einer Selektivität von 84 %.

### Beispiel 17: Umsetzung 4-tert-Butvlphenyl-N-Phenylcarbamat zu Phenylisocyanat durch Spaltung bei 200 °C in Gegenwart von Natriumhydrogensulfat (Katalysator des Typs (C)) bei einem molaren Verhältnis von Carbamat zu Katalysator von 15,3 : 1

In einem inertisierten Mehrhalskolben wurden 0,64 g (3,59 mmol) Phenanthren sowie 0,26 g (2,17 mmol) Natriumhydrogensulfat in 30,16 g (177,19 mmol) Diphenylether gelöst. Das Reaktionsgemisch wurde auf 215 °C erwärmt. In einem inertisierten Schlenkrohr wurden 8,94 g (33,19 mmol) 4-tert-Butylphenyl-N-Phenylcarbamat auf 150 °C erwärmt. Durch das vollständige Überführen des 4-tert-Butylphenyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels HPLC Chromatographie verfolgt.

Die Ausbeute an Phenylisocyanat betrug 48 % mit einer Selektivität von 74 %.

### Beispiel 18: Umsetzung von 4-tert-Butylphenyl-N-Phenylcarbamat zu Phenylisocyanat durch thermische Spaltung bei 200 °C in Diphenylether (Vergleichsbeispiel zu Beispiel 17)

In einem inertisierten Mehrhalskolben wurden 0,60 g (3,37 mmol) Phenanthren in 31,26 g (183,67 mmol) Diphenylether gelöst. Das Reaktionsgemisch wurde auf 215 °C erwärmt. In einem inertisierten Schlenkrohr wurden 8,76 g (32,52 mmol) 4-tert-Butylphenyl-N-Phenylcarbamat auf 150 °C erwärmt. Durch das vollständige Überführen des 4-tert-Butylphenyl-N-Phenylcarbamat in das Reaktionsgemisch resultierte ein Gemisch mit einer Temperatur von 200 °C. Diese Temperatur wurde für 120 Minuten konstant gehalten. Die entstehenden gasförmigen Reaktionsprodukte wurden bei einem Argon-Inertgasstrom von 10 L/h ausgetrieben und in einer Kühlfalle gesammelt. Der Reaktionsfortschritt wurde durch kontinuierliche Probennahme aus dem Reaktionsgefäß und anschließender Analyse mittels HPLC Chromatographie verfolgt.

Die Ausbeute an Phenylisocyanat betrug 23 % mit einer Selektivität von 87 %.

## Patentansprüche

1. Verfahren zur Herstellung eines Isocyanats, bei welchem ein Carbamat oder Thiolcarbamat in Gegenwart eines Katalysators unter Abspaltung eines Alkohols oder Thioalkohols bei einer Temperatur von mindestens 150 °C zu dem korrespondierenden Isocyanat umgesetzt wird,
**dadurch gekennzeichnet, dass** als Katalysator eine Verbindung der allgemeinen Formel (X)(Y)(Z-H) eingesetzt wird, wobei gilt:
(A)
• X steht für N(R¹),
• Y steht für C(R²) oder für eine Brücke aus 2 Kohlenstoffatomen, die Bestandteil eines Ringsystems aus 5 oder 7 Kohlenstoffatomen mit alternierenden Doppel- und Einfachbindungen sind, oder für eine Brücke aus 3, 5 oder 7 Kohlenstoffatomen mit alternierenden Einfach- und Doppelbindungen, und
• Z steht für O, S, N(R⁶) oder N⁽⁺⁾(R⁷)(R⁸),
• wobei die Katalysatoren des Typs (A) einen pK_{B}-Wert bei 25 °C von ≥ 3,00 aufweisen;
oder (B)
• X steht für O,
• Y steht für C(R²) oder für eine Brücke aus 2 Kohlenstoffatomen, die Bestandteil eines Ringsystems aus 5 oder 7 Kohlenstoffatomen mit alternierenden Doppel- und Einfachbindungen sind, oder für eine Brücke aus 3, 5 oder 7 Kohlenstoffatomen mit alternierenden Einfach- und Doppelbindungen, und
• Z steht für O;
oder (C)
• X steht für O,
• Y steht für S(O)(R³) oder P(OR⁴)(OR⁵), und
• Z steht für O;
wobei gilt:
R¹ ist
• ein, optional substituierter, aromatischer oder araliphatischer Rest mit 6 bis 10 Kohlenstoffatomen oder
• ein, optional substituierter, aliphatischer Rest mit 1 bis 6 Kohlenstoffatomen oder
• mit R² oder R⁸ zu einem aus insgesamt 5 bis 8 Atomen bestehenden Ring verbunden, wobei der Ring optional Heteroatome, insbesondere Stickstoff und/oder Schwefel, umfassen kann;
R² ist
• Wasserstoff oder
• ein, optional substituierter, aromatischer oder araliphatischer Rest mit 6 bis 10 Kohlenstoffatomen oder
• ein, optional substituierter und optional Ethereinheiten aufweisender, aliphatischer Rest mit 1 bis 6 Kohlenstoffatomen oder
• mit R¹ oder R⁶ oder R⁷ zu einem aus insgesamt 5 bis 8 Atomen bestehenden Ring verbunden, wobei der Ring optional Heteroatome, insbesondere Stickstoff und/oder Schwefel, umfassen kann;
R³ ist
• ein mit einer Sulfonsäure- oder Sulfonatgruppe substituierter, aromatischer oder araliphatischer Rest mit 6 bis 10 Kohlenstoffatomen oder
• ein mit einer Amingruppe, Sulfonsäure- oder Sulfonatgruppe substituierter, aliphatischer Rest mit 1 bis 6 Kohlenstoffatomen oder
• O⁽⁻⁾M⁽⁺⁾, wobei M⁽⁺⁾
für ein Alkalimetallkation, Imidazoliumkation, Pyridiniumkation, Pyrrolidiniumkation, Phosphoniumkation, Sulfoniumkation oder NH₄⁺, steht,
oder für ein mono-, di-, tri- oder tetra-substituiertes organisches Ammoniumkation, dessen organische Substituenten unabhängig von einander ausgewählt sind aus der Gruppe bestehend aus Methyl, Ethyl, Propyl, Butyl, Pentyl, Hexyl, Phenyl und Cyclohexyl, steht,
wobei besonders bevorzugt M⁽⁺⁾ für ein Alkalimetallkation oder NH₄⁺, ganz besonders bevorzugt für ein Alkalimetallkation ausgewählt aus Li⁺, Na⁺ oder K⁺, steht;
R⁴ und R⁵ sind unabhängig voneinander
• optional subsitituierte, aromatische oder araliphatische Reste mit jeweils 6 bis 10 Kohlenstoffatomen, wobei R⁴ und R⁵ zu einem aus 5 bis 8 Atomen bestehenden Ring verbunden sein können, oder
• optional subsitituierte, aliphatische Reste mit jeweils 1 bis 6 Kohlenstoffatomen, wobei R⁴ und R⁵ zu einem aus 5 bis 8 Atomen bestehenden Ring verbunden sein können;
R⁶ ist
• ein, optional substituierter, aromatischer oder araliphatischer Rest mit 6 bis 10 Kohlenstoffatomen oder
• ein, optional substituierter, aliphatischer Rest mit 1 bis 6 Kohlenstoffatomen oder
• mit R² zu einem aus insgesamt 5 bis 8 Atomen bestehenden Ring verbunden, wobei der Ring optional Heteroatome, insbesondere Stickstoff und/oder Schwefel, umfassen kann;
R⁷ ist
• ein, optional substituierter, aromatischer oder araliphatischer Rest mit 6 bis 10 Kohlenstoffatomen oder
• ein, optional substituierter, aliphatischer Rest mit 1 bis 6 Kohlenstoffatomen oder
• mit R² zu einem aus insgesamt 5 bis 8 Atomen bestehenden Ring verbunden, wobei der Ring optional Heteroatome, insbesondere Stickstoff und/oder Schwefel, umfassen kann;
R⁸ ist
• ein, optional substituierter, aromatischer oder araliphatischer Rest mit 6 bis 10 Kohlenstoffatomen oder
• ein, optional substituierter, aliphatischer Rest mit 1 bis 6 Kohlenstoffatomen oder
• mit R¹ zu einem aus insgesamt 5 bis 8 Atomen bestehenden Ring verbunden.

2. Verfahren gemäß Anspruch 1, bei welchem die Umsetzung des Ausgangs-Carbamats bzw. -Thiolcarbamats in Lösung in Gegenwart eines organischen Lösungsmittels ausgewählt aus aprotischen polaren Lösungsmittel ohne Isocyanat-reaktive Gruppen durchgeführt wird.

3. Verfahren gemäß Anspruch 2, bei welchem eine Konzentration des Ausgangs-Carbamats bzw. -Thiolcarbamats in der Lösung im Bereich von 5 Massen-% bis 95 Massen-%, bezogen auf die Gesamtmasse der Lösung, eingestellt wird.

4. Verfahren gemäß einem der vorstehenden Ansprüche, bei welchem ein molares Verhältnis von Ausgangs-Carbamat bzw. -Thiolcarbamat zu Katalysator von 1000 : 1 bis 1 : 1 eingesetzt wird.

5. Verfahren gemäß einem der vorstehenden Ansprüche, bei welchem die Umsetzung bei einer Temperatur im Bereich von 150 °C bis 280 °C und bei einem Druck im Bereich von 0,001 bar_{(abs.)} bis 2,00 bar_{(abs.)} durchgeführt wird.

6. Verfahren gemäß einem der vorstehenden Ansprüche, bei welchem das gebildete Isocyanat und/oder der gebildete Alkohol bzw. Thioalkohol kontinuierlich oder in Intervallen aus dem Reaktionsgemisch entfernt wird/werden.

7. Verfahren gemäß Anspruch 6, bei welchem der gebildete Alkohol bzw. Thioalkohol kontinuierlich oder in Intervallen aus dem Reaktionsgemisch entfernt wird, wobei das Entfernen des Alkohols bzw. Thioalkohols durch Durchleiten eines Stripgases und/oder destillativ bewirkt wird, optional unterstützt durch Anlegen eines gegenüber Umgebungsdruck verminderten Drucks.

8. Verfahren gemäß Anspruch 6, bei welchem das gebildete Isocyanat und der gebildete Alkohol bzw. Thioalkohol kontinuierlich oder in Intervallen aus dem Reaktionsgemisch entfernt werden, wobei (i) entweder beide zusammen entfernt werden, gefolgt von einer Trennung des anfallenden gasförmigen Gemisches enthaltend das Isocyanat und den Alkohol bzw. Thioalkohol durch fraktionierende Kondensation, oder (ii) zuerst der Alkohol bzw. Thioalkohol und danach das Isocyanat entfernt wird.

9. Verfahren gemäß Anspruch 8, bei welchem zur Entfernung des Alkohols bzw. Thioalkohols und des Isocyanats das Reaktionsgemisch kontinuierlich in zwei hintereinandergeschalteten Destillationskolonnen destilliert wird.

10. Verfahren gemäß einem der vorstehenden Ansprüche, bei welchem das herzustellende Isocyanat
1,4-Butylendiisocyanat, 1,5-Pentandiisocyanat, 1,6-Hexamethylendiisocyanat oder deren Dimere, Trimere, Pentamere, Heptamere oder Nonamere oder Gemische derselben, Isophorondiisocyanat, 2,2,4- und/oder 2,4,4-Trimethylhexamethylendiisocyanat, die isomeren Bis(4,4'-isocyanatocyclohexyl)methane oder deren Mischungen in beliebigen Anteilen, 1,4-Cyclohexylendiisocyanat, Phenylisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat und/oder dessen höhere Homologe, 1,3- und/oder 1,4-Bis-(2-isocyanato-prop-2-yl)-benzol, 1,3-Bis-(isocyanatomethyl)benzol, oder ein Alkyl-2,6-diisocyanatohexanoat (Lysindiisocyanat) mit Alkylgruppen aus 1 Kohlenstoffatom bis 6 Kohlenstoffatomen,
ist, wobei als Carbamat bzw. Thiolcarbamat das zu dem herzustellenden Isocyanant korrespondierende
Methyl-, Ethyl-, *n*-Propyl-, *iso*-Propyl-, *n*-Butyl-, *iso*-Butyl-, *tert*-Butyl-, Cyclohexyl- oder Phenyl-Carbamat bzw. -Thiolcarbamat
oder
substituierte Methyl-, Ethyl-, *n*-Propyl-, *iso-*Propyl-, *n*-Butyl-, *iso*-Butyl-, *tert*-Butyl*-,* Cyclohexyl- oder Phenyl-Carbamat bzw. -Thiolcarbamat
eingesetzt wird.

11. Verfahren gemäß einem der vorstehenden Ansprüche, bei welchem neben dem Katalysator (X)(Y)(Z)H kein weiterer Katalysator eingesetzt wird.

12. Verfahren gemäß einem der vorstehenden Ansprüche, bei welchem der Katalysator (X)(Y)(Z-H) ausgewählt ist aus der Gruppe bestehend aus 2-Hydroxy-2,4,6-cycloheptatrien-1-on (Tropolon) **(a);** ; N,N'-Diphenylformamidin **(b);** *N*-(2,6-Dimethylphenyl)-5,6-dihydro-4*H*-1,3-thiazin-2-amin (Xylazin) **(c);** 2,3-Dihydro-7-azaindol **(d);** protoniertes N-Methyl-1,5,7-triazabicyclo[4.4.0]dec-5-en **(e);** protoniertes 1,8-Diazabicyclo[5.4.0]undec-7-en **(f);** O-Methyl-N,N'-diisopropylisoharnstoff **(g);** 2-Mercaptopyridin **(h);** 1,3,4-Thiadiazol-2,5-dithiol (i); Mercaptobenzimidazol **(j);** den Konstitutionsisomeren des Benzoldisulfonsäure-Monoanions **(k);** den Konstitutionsisomeren der Benzoldisulfonsäure **(l)**; (R)-(-)-1,1'-Binaphthyl-2,2'-hydrogenphosphat (**m**); Dibenzylhydrogenphosphat **(n);** 2,6-Naphtalindisulfonsäure-Monoanion **(o);** Alkalimetallhydrogensulfat **(p),** 2-Aminoethan-1-Sulfonsäure (Taurin) **(q)** und Mischungen **(r)** der vorgenannten Verbindungen.

13. Verfahren gemäß einem der Ansprüche 1 bis 12, insbesondere gemäß Anspruch 12, bei welchem ein Katalysator des Typs (A) eingesetzt wird.

14. Verfahren gemäß einem der Ansprüche 1 bis 12, insbesondere gemäß Anspruch 12, bei welchem ein Katalysator des Typs (B) eingesetzt wird.

15. Verfahren gemäß einem der Ansprüche 1 bis 12, insbesondere gemäß Anspruch 12, bei welchem ein Katalysator des Typs (C) eingesetzt wird.

## Claims

1. Process for producing an isocyanate in which a carbamate or thiolcarbamate is converted into the corresponding isocyanate in the presence of a catalyst with elimination of an alcohol or thioalcohol at a temperature of at least 150°C,
**characterized in that** the catalyst used is a compound of the general formula (X) (Y) (Z-H), where:
(A)
• X is N (R¹),
• Y is C(R²) or is a bridge formed of 2 carbon atoms which are part of a ring system composed of 5 or 7 carbon atoms with alternating double and single bonds, or is a bridge formed of 3, 5 or 7 carbon atoms with alternating single and double bonds, and
• Z is O, S, N (R⁶) or N⁽⁺⁾ (R⁷) (R⁸),
• wherein the catalysts of type (A) have a pK_{B} at 25°C of ≥ 3.00;
or (B)
• X is O,
• Y is C(R²) or is a bridge formed of 2 carbon atoms which are part of a ring system composed of 5 or 7 carbon atoms with alternating double and single bonds, or is a bridge formed of 3, 5 or 7 carbon atoms with alternating single and double bonds, and
• Z is O;
or (C)
• X is O,
• Y is S(O)(R³) or P(OR⁴)(OR⁵), and
• Z is O;
where:
R¹ is
• an optionally substituted aromatic or araliphatic radical having 6 to 10 carbon atoms or
• an optionally substituted aliphatic radical having 1 to 6 carbon atoms or
• joined to R² or R⁸ to form a ring consisting of a total of 5 to 8 atoms, wherein the ring can optionally comprise heteroatoms, especially nitrogen and/or sulfur;
R² is
• hydrogen or
• an, optionally substituted, aromatic or araliphatic radical having 6 to 10 carbon atoms or
• an, optionally substituted, aliphatic radical having 1 to 6 carbon atoms and optionally comprising ether units or
• joined to R¹ or R⁶ or R⁷ to form a ring consisting of a total of 5 to 8 atoms, wherein the ring can optionally comprise heteroatoms, especially nitrogen and/or sulfur;
R³ is
• an aromatic or araliphatic radical having 6 to 10 carbon atoms which is substituted by a sulfonic acid group or sulfonate group or
• an aliphatic radical having 1 to 6 carbon atoms which is substituted by an amine group, sulfonic acid group or sulfonate group or
• O⁽⁻⁾M⁽⁺⁾, where M⁽⁺⁾
is an alkali metal cation, imidazolium cation, pyridinium cation, pyrrolidinium cation, phosphonium cation, sulfonium cation or NH₄⁺,
or is a mono-, di-, tri- or tetrasubstituted organic ammonium cation the organic substituents of which independently of one another are selected from the group consisting of methyl, ethyl, propyl, butyl, pentyl, hexyl, phenyl and cyclohexyl,
where M⁽⁺⁾ particularly preferably is an alkali metal cation or NH₄⁺, very particularly preferably is an alkali metal cation selected from Li⁺, Na⁺ or K⁺;
R⁴ and R⁵ independently of one another are
• optionally substituted aromatic or araliphatic radicals each having 6 to 10 carbon atoms, where R⁴ and R⁵ may be joined to form a ring consisting of 5 to 8 atoms, or
• optionally substituted aliphatic radicals each having 1 to 6 carbon atoms, where R⁴ and R⁵ may be joined to form a ring consisting of 5 to 8 atoms;
R⁶ is
• an optionally substituted aromatic or araliphatic radical having 6 to 10 carbon atoms or
• an optionally substituted aliphatic radical having 1 to 6 carbon atoms or
• joined to R² to form a ring consisting of a total of 5 to 8 atoms, wherein the ring can optionally comprise heteroatoms, especially nitrogen and/or sulfur;
R⁷ is
• an optionally substituted aromatic or araliphatic radical having 6 to 10 carbon atoms or
• an optionally substituted aliphatic radical having 1 to 6 carbon atoms or
• joined to R² to form a ring consisting of a total of 5 to 8 atoms, wherein the ring can optionally comprise heteroatoms, especially nitrogen and/or sulfur;
R⁸ is
• an optionally substituted aromatic or araliphatic radical having 6 to 10 carbon atoms or
• an optionally substituted aliphatic radical having 1 to 6 carbon atoms or
• joined to R¹ to form a ring consisting of a total of 5 to 8 atoms.

2. Process according to Claim 1, in which the conversion of the starting carbamate or starting thiolcarbamate is conducted in solution in the presence of an organic solvent selected from aprotic polar solvents without isocyanate-reactive groups.

3. Process according to Claim 2, in which a concentration of the starting carbamate or starting thiolcarbamate in the solution is set in the range from 5% by mass to 95% by mass, based on the total mass of the solution.

4. Process according to any of the preceding claims, in which a molar ratio of starting carbamate or starting thiolcarbamate to catalyst of 1000:1 to 1:1 is used.

5. Process according to any of the preceding claims, in which the conversion is conducted at a temperature in the range from 150°C to 280°C and at a pressure in the range from 0.001 bar_{(abs.)} to 2.00 bar_{(abs.)}.

6. Process according to any of the preceding claims, in which the isocyanate formed and/or the alcohol or thioalcohol formed is/are removed from the reaction mixture continuously or at intervals.

7. Process according to Claim 6, in which the alcohol or thioalcohol formed is removed from the reaction mixture continuously or at intervals, wherein the removal of the alcohol or thioalcohol is effected by passing through a stripping gas and/or by distillation, optionally assisted by application of a pressure which is reduced compared to ambient pressure.

8. Process according to Claim 6, in which the isocyanate formed and the alcohol or thioalcohol formed are removed from the reaction mixture continuously or at intervals, wherein (i) either both are removed together, followed by a separation of the gaseous mixture obtained containing the isocyanate and the alcohol or thioalcohol by means of fractional condensation, or (ii) first the alcohol or thioalcohol and then the isocyanate is removed.

9. Process according to Claim 8, in which, for the removal of the alcohol or thioalcohol and of the isocyanate, the reaction mixture is distilled continuously in two series-connected distillation columns.

10. Process according to any of the preceding claims, in which the isocyanate to be produced is
butylene 1,4-diisocyanate, pentane 1,5-diisocyanate, hexamethylene 1,6-diisocyanate or the dimers, trimers, pentamers, heptamers or nonamers thereof or mixtures of same, isophorone diisocyanate, 2,2,4- and/or 2,4,4-trimethylhexamethylene diisocyanate, the isomeric bis(4,4'-isocyanatocyclohexyl)methanes or mixtures thereof in any desired proportions, cyclohexylene 1,4-diisocyanate, phenyl isocyanate, phenylene 1,4-diisocyanate, tolylene 2,4- and/or 2,6-diisocyanate, naphthylene 1,5-diisocyanate, diphenylmethane 2,2'- and/or 2,4'- and/or 4,4'-diisocyanate and/or the higher homologs thereof, 1,3- and/or 1,4-bis(2-isocyanatoprop-2-yl)benzene, 1,3-bis(isocyanatomethyl)benzene, or an alkyl 2,6-diisocyanatohexanoate (lysine diisocyanate) having alkyl groups of 1 carbon atom to 6 carbon atoms, wherein the carbamate or thiolcarbamate used is the methyl, ethyl, n-propyl, isopropyl, n-butyl, isobutyl, tert-butyl, cyclohexyl or phenyl carbamate or thiolcarbamate
or
substituted methyl, ethyl, n-propyl, isopropyl, n-butyl, isobutyl, tert-butyl, cyclohexyl or phenyl carbamate or thiolcarbamate
which corresponds to the isocyanate to be produced.

11. Process according to any of the preceding claims, in which no further catalyst is used besides the catalyst (X) (Y) (Z)H.

12. Process according to any of the preceding claims, in which the catalyst (X) (Y) (Z-H) is selected from the group consisting of 2-hydroxy-2,4,6-cycloheptatrien-1-one (tropolone) (a); N,N'-diphenylformamidine (b); N-(2,6-dimethylphenyl)-5,6-dihydro-4H-1,3-thiazin-2-amine (xylazine) (c); 2,3-dihydro-7-azaindole (d); protonated N-methyl-1,5,7-triazabicyclo[4.4.0]dec-5-ene (e); protonated 1,8-diazabicyclo[5.4.0]undec-7-ene (f); O-methyl-N,N'-diisopropylisourea (g); 2-mercaptopyridine (h); 1,3,4-thiadiazole-2,5-dithiol (i); mercaptobenzimidazole (j); the constitutional isomers of the benzenedisulfonic acid monoanion (k); the constitutional isomers of benzenedisulfonic acid (1); (R)-(-)-1,1'-binaphthyl-2,2'-hydrogenphosphate (m); dibenzyl hydrogenphosphate (n); naphthalene-2,6-disulfonic acid monoanion (o); alkali metal hydrogensulfate (p), 2-aminoethane-1-sulfonic acid (taurine) (q) and mixtures (r) of the compounds mentioned.

13. Process according to any of Claims 1 to 12, especially according to Claim 12, in which a catalyst of type (A) is used.

14. Process according to any of Claims 1 to 12, especially according to Claim 12, in which a catalyst of type (B) is used.

15. Process according to any of Claims 1 to 12, especially according to Claim 12, in which a catalyst of type (C) is used.

## Revendications

1. Procédé pour la préparation d'un isocyanate, dans lequel un carbamate ou un thiolcarbamate est transformé en présence d'un catalyseur avec dissociation d'un alcool ou d'un thioalcool à une température d'au moins 150°C en isocyanate correspondant, **caractérisé en ce qu'**on utilise, comme catalyseur, un composé de formule générale (X) (Y) (Z-H), dans laquelle :
(A)
X représente N(R¹),
Y représente C(R²) ou un pont de 2 atomes de carbone, qui font partie d'un système cyclique constitué par 5 ou 7 atomes de carbone présentant des doubles liaisons et des simples liaisons en alternance ou un pont constitué par 3, 5 ou 7 atomes de carbone présentant des simples liaisons et des doubles liaisons en alternance et
Z représente O, S, N(R⁶) ou N⁽⁺⁾(R⁷)(R⁸),
les catalyseurs du type (A) présentant une valeur pK_{B} à 25°C ≥ 3,00 ; ou
(B)
X représente O,
Y représente C(R²) ou un pont de 2 atomes de carbone, qui font partie d'un système cyclique constitué par 5 ou 7 atomes de carbone présentant des doubles liaisons et des simples liaisons en alternance ou un pont constitué par 3, 5 ou 7 atomes de carbone présentant des simples liaisons et des doubles liaisons en alternance et
Z représente O ou
(C)
X représente O,
Y représente S(O)(R³) ou P(OR⁴)(OR⁵) et
Z représente O ;
où :
R¹
- représente un radical aromatique ou araliphatique, éventuellement substitué, comprenant 6 à 10 atomes de carbone ou
- représente un radical aliphatique, éventuellement substitué, comprenant 1 à 6 atomes de carbone ou
- est relié avec R² ou R⁸ en un cycle constitué par au total 5 à 8 atomes de carbone, le cycle pouvant comprendre éventuellement des hétéroatomes, en particulier azote et/ou soufre ;
R2
- représente hydrogène ou
- représente un radical aromatique ou araliphatique, éventuellement substitué, comprenant 6 à 10 atomes de carbone ou
- représente un radical aliphatique, éventuellement substitué et présentant éventuellement des motifs éther, comprenant 1 à 6 atomes de carbone ou
- est relié avec R¹ ou R⁶ ou R⁷ en un cycle constitué par au total 5 à 8 atomes de carbone, le cycle pouvant comprendre éventuellement des hétéroatomes, en particulier azote et/ou soufre ;
R³ représente
- un radical aromatique ou araliphatique, substitué par un groupe acide sulfonique ou sulfonate, comprenant 6 à 10 atomes de carbone ou
- un radical aliphatique, substitué par un groupe amine, un groupe acide sulfonique ou sulfonate, comprenant 1 à 6 atomes de carbone ou
- O⁽⁻⁾M⁽⁺⁾, où M⁽⁺⁾
représente un cation de métal alcalin, un cation d'imidazolium, un cation de pyridinium, un cation de pyrrolidinium, un cation de phosphonium, un cation de sulfonium ou NH₄⁺,
ou un cation d'ammonium organique monosubstitué, disubstitué, trisubstitué ou tétrasubstitué, dont les substituants organiques sont choisis, indépendamment les uns des autres, dans le groupe constitué par méthyle, éthyle, propyle, butyle, pentyle, hexyle, phényl et cyclohexyle,
M⁽⁺⁾ représentant de manière particulièrement préférée un cation de métal alcalin ou NH₄⁺, de manière tout particulièrement préférée un cation de métal alcalin choisi parmi Li⁺, Na⁺ ou K⁺ ;
R⁴ et R⁵ représentent, indépendamment l'un de l'autre,
- des radicaux aromatiques ou araliphatiques, éventuellement substitués, comprenant à chaque fois 6 à 10 atomes de carbone, R⁴ et R⁵ pouvant être reliés en un cycle constitué par 5 à 8 atomes de carbone, ou
- des radicaux aliphatiques, éventuellement substitués, comprenant à chaque fois 1 à 6 atomes de carbone, R⁴ et R⁵ pouvant être reliés en un cycle constitué par 5 à 8 atomes de carbone ;
R6
- représente un radical aromatique ou araliphatique, éventuellement substitué, comprenant 6 à 10 atomes de carbone ou
- représente un radical aliphatique, éventuellement substitué, comprenant 1 à 6 atomes de carbone ou
- est relié avec R² en un cycle constitué par au total 5 à 8 atomes de carbone, le cycle pouvant comprendre éventuellement des hétéroatomes, en particulier azote et/ou soufre ;
R7
- représente un radical aromatique ou araliphatique, éventuellement substitué, comprenant 6 à 10 atomes de carbone ou
- représente un radical aliphatique, éventuellement substitué, comprenant 1 à 6 atomes de carbone ou
- est relié avec R² en un cycle constitué par au total 5 à 8 atomes de carbone, le cycle pouvant comprendre éventuellement des hétéroatomes, en particulier azote et/ou soufre ;
R⁸
- représente un radical aromatique ou araliphatique, éventuellement substitué, comprenant 6 à 10 atomes de carbone ou
- représente un radical aliphatique, éventuellement substitué, comprenant 1 à 6 atomes de carbone ou
- est relié avec R¹ en un cycle constitué par au total 5 à 8 atomes de carbone.

2. Procédé selon la revendication 1, dans lequel la transformation du carbamate ou du thiolcarbamate de départ est réalisée en solution en présence d'un solvant organique choisi parmi les solvants polaires aprotiques sans groupes réactifs vis-à-vis d'isocyanate.

3. Procédé selon la revendication 2, dans lequel une concentration en carbamate ou en thiolcarbamate de départ dans la solution est réglée dans la plage de 5% en masse à 95% en masse, par rapport à la masse totale de la solution.

4. Procédé selon l'une quelconque des revendications précédentes, dans lequel un rapport molaire de carbamate ou de thiolcarbamate de départ à catalyseur de 1000:1 à 1:1 est utilisé.

5. Procédé selon l'une quelconque des revendications précédentes, dans lequel la transformation est réalisée à une température dans la plage de 150°C à 280°C et à une pression dans la plage de 0,001 bar_{(abs)} à 2,00 bars_{(abs)}.

6. Procédé selon l'une quelconque des revendications précédentes, dans lequel l'isocyanate formé et/ou l'alcool ou le thioalcool formé est/sont éliminé(s) en continu ou à intervalles à partir du mélange réactionnel.

7. Procédé selon la revendication 6, dans lequel l'alcool ou le thioalcool formé est éliminé en continu ou à intervalles à partir du mélange réactionnel, l'élimination de l'alcool ou du thioalcool étant provoquée par le passage d'un gaz d'extraction et/ou par distillation, éventuellement soutenu(e) par application d'une pression réduite par rapport à la pression ambiante.

8. Procédé selon la revendication 6, dans lequel l'isocyanate formé et l'alcool ou le thioalcool formé sont éliminés en continu ou à intervalles à partir du mélange réactionnel, soit (i) les deux étant éliminés ensemble, l'élimination étant suivie d'une séparation du mélange gazeux formé contenant l'isocyanate et l'alcool ou le thioalcool par condensation fractionnée, soit (ii) d'abord l'alcool ou le thioalcool, puis l'isocyanate étant éliminés.

9. Procédé selon la revendication 8, dans lequel, pour l'élimination de l'alcool ou du thioalcool et de l'isocyanate, le mélange réactionnel est distillé en continu dans deux colonnes de distillation successives.

10. Procédé selon l'une quelconque des revendications précédentes, dans lequel l'isocyanate à préparer est le diisocyanate de 1,4-butylène, le diisocyanate de 1,5-pentane, le diisocyanate de 1,6-hexaméthylène ou leur dimères, trimères, pentamères, heptamères ou nonamères ou meurs mélanges, le diisocyanate d'isophorone, le diisocyanate de 2,2,4-triméthylhexaméthylène et/ou de 2,4,4-triméthylhexaméthylène, les bis(4,4'-isocyanatocyclohexyl)méthanes isomères ou leurs mélanges en des proportions quelconques, le diisocyanate de 1,4-cyclohexylène, l'isocyanate de phényle, le diisocyanate de 1,4-phénylène, le diisocyanate de 2,4-toluylène et/ou de 2,6-toluylène, le diisocyanate de 1,5-naphtylène, le diisocyanate de 2,2'-diphénylméthane et/ou de 2,4'-diphénylméthane et/ou de 4,4'-diphénylméthane et/ou leurs homologues supérieurs, le 1,3-bis-(2-isocyanatoprop-2-yl)-benzène et/ou le 1,4-bis-(2-isocyanatoprop-2-yl)-benzène, le 1,3-bis-(isocyanatométhyl)benzène ou un 2,6-diisocyanatohexanoate d'alkyle (diisocyanate de lysine) présentant des groupes alkyle constitués par 1 atome de carbone à 6 atomes de carbone, où on utilise, comme carbamate ou thiolcarbamate, le carbamate ou le thiolcarbamate de méthyle, d'éthyle, de n-propyle, d'iso-propyle, de n-butyle, d'iso-butyle, de tert-butyle, de cyclohexyle ou de phényle ou le carbamate ou le thiolcarbamate de méthyle, d'éthyle, de n-propyle, d'iso-propyle, de n-butyle, d'iso-butyle, de tert-butyle, de cyclohexyle ou de phényle substitué, correspondant à l'isocyanate à préparer.

11. Procédé selon l'une quelconque des revendications précédentes, dans lequel on n'utilise pas d'autre catalyseur en plus du catalyseur (X)(Y)(Z)H.

12. Procédé selon l'une quelconque des revendications précédentes, dans lequel le catalyseur (X) (Y) (Z-H) est choisi dans le groupe constitué par la 2-hydroxy-2,4,6-cycloheptatrién-1-one (tropolone) (a) ; la N,N'-diphénylformamidine (b) ; la N-(2,6-diméthylphényl)-5,6-dihydro-4H-1,3-thiazin-2-amine (xylazine) (c) ; le 2,3-dihydro-7-aza-indole (d) ; le N-méthyl-1,5,7-triazabicyclo[4,4,0]déc-5-ène protoné (e) ; le 1,8-diazabicyclo[5,4,0]undéc-7-ène protoné (f) ; l'O-méthyl-N,N'-diisopropyliso-urée (g) ; la 2-mercaptopyridine (h) ; le 1,3,4-thiadiazol-2,5-dithiol (i) ; le mercaptobenzimidazole (j) ; les isomères de constitution du mono-anion de l'acide benzènedisulfonique (k) ; les isomères de constitution de l'acide benzènedisulfonique (1) ; le 2,2'-hydrogénophosphate de (R)-(-)-1,1'-binaphtyle (m) ; l'hydrogénophosphate de dibenzyle (n) ; le mono-anion de l'acide 2,6-naphtalènedisulfonique (o) ; l'hydrogénosulfate de métal alcalin (p) ; l'acide 2-aminoéthane-1-sulfonique (taurine) ; (q) et les mélanges (r) des composés susmentionnés.

13. Procédé selon l'une quelconque des revendications 1 à 12, en particulier selon la revendication 12, dans lequel un catalyseur du type (A) est utilisé.

14. Procédé selon l'une quelconque des revendications 1 à 12, en particulier selon la revendication 12, dans lequel un catalyseur du type (B) est utilisé.

15. Procédé selon l'une quelconque des revendications 1 à 12, en particulier selon la revendication 12, dans lequel un catalyseur du type (C) est utilisé.
